# EUROPEAN PATENT SPECIFICATION

(11) **EP 0 642 503 B1**
(45) Date of publication and mention of the grant of the patent: **01.12.1999**
(21) Application number: 92920702.5
(22) Date of filing: 22.09.1992
(51) Int. Cl.: C07D 305/14, A61K 31/335

(54) **10-DESACETOXYTAXOL DERIVATIVES**
10-DISACETOXYTAXOLDERIVATE
DERIVES DE 10-DESACETOXYTAXOL

(30) Priority: 23.09.1991 US 763805; 18.06.1992 US 900408
(43) Date of publication of application: 15.03.1995
(73) Proprietor: FLORIDA STATE UNIVERSITY, Tallahassee, Florida 32310 (US); BRISTOL-MYERS SQUIBB COMPANY, New York, N.Y. 10154-0037 (US)
(72) Inventor: HOLTON, Robert, A. Florida State University, Room 715 Tallahassee, FL 32306 (US); CHEN, Shu-Hui Bristol-Myers Squibb Company,, Wallingford, CT 06492-7660 (US); FARINA, Vittorio Bristol-Myers Squibb Company,, Wallingford, CT 06492-7660 (US)
(74) Representative: Eyles, Christopher Thomas
(86) International application number: US9207935
(87) International publication number: WO9306093

(56) References cited:
- J. Org. Chem., 1991, KINGSTON et al., "Modified Taxols. Reaction of Taxol with Electrophilic Reagents and Preparation of a Rearranged Taxol Derivative with Tubulin Assembly Activity", Vol. 56, No. 17, pp. 5114 to 5119.

## Description

### BACKGROUND OF THE INVENTION

The present invention relates to novel antitumor agents. More particularly, the present invention relates to 10-desacetoxytaxol and derivatives thereof.

Taxol is a natural product extracted from the bark of yew trees. It has been shown to have excellent antitumor activity in in vivo animal models, and recent studies have elucidated its unique mode of action, which involves abnormal polymerization of tubulin and disruption of mitosis. It is currently undergoing clinical trials against ovarian, breast and other types of cancer in the United States and France and preliminary results have confirmed it as a most promising chemotherapeutic agent. The structure of taxol and the numbering system conventionally used is shown below; this numbering system is also applicable to compounds of the present invention.

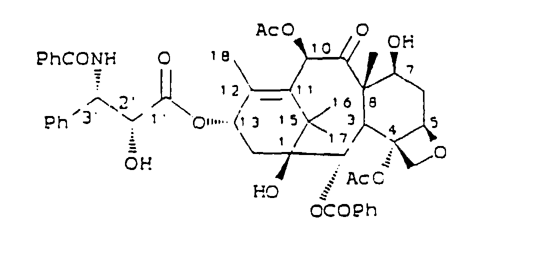

### SUMMARY OF THE INVENTION

Among the objects of the present invention, therefore, is the provision of the novel taxane, 10-desacetoxytaxol, and derivatives thereof, which exhibit antitumor activity.

In accordance with the present invention, a compound is provided having the following structural formula (1): 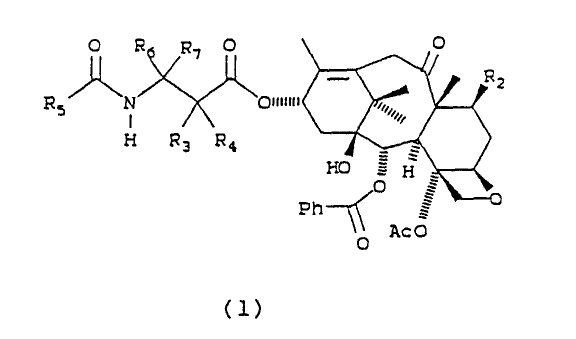 wherein R₂ is hydrogen, hydroxy or a protected hydroxy group; R₃ and R₄ are independently hydrogen, hydroxy, a protected hydroxy group, methyl, -SH, -NH₂, or -NR₈R₉; R₅ is R₁₀, or -OR₁₀; R₆ and R₇ are independently hydrogen, alkyl, or aryl; R₈ and R₉ are independently hydrogen, alkyl, alkenyl, alkynyl, or aryl; and R₁₀ is, alkyl, alkenyl, alkynyl, or aryl; provided that when R₂ is hydrogen, R₃ and R₄ are independently hydrogen, methyl, -SH, -NH₂, or -NR₈R₉.

Also provided are the compounds 10-desacetoxytaxol and 10-deoxytaxotere.

Other objects and features of this invention will be in part apparent and in part pointed out hereinafter.

### DETAILED DESCRIPTION OF THE INVENTION

As used herein "Ar" means aryl; "Ph" means phenyl; "Ac" means acetyl; "R" means alkyl; "ll-ene" means a double bond between carbon atoms 11 and 12 of a compound of formula (1); and "10,12-diene" means double bonds between carbon atoms 10 and 11, and 12 and 18 of a compound of formula (1). "Hydroxy protecting group" includes, but is not limited to, ethers such as methyl, t-butyl, benzyl, p-methoxybenzyl, p-nitrobenzyl, allyl, trityl, methoxymethyl, methoxyethoxymethyl, ethoxyethyl, tetrahydropyranyl, tetrahydrothiopyranyl, and trialkylsilyl ethers such as trimethylsilyl ether, triethylsilyl ether, dimethylarylsilyl ether, triisopropylsilyl ether and t-butyldimethylsilyl ether; esters such as benzoyl, acetyl, phenylacetyl, formyl, mono-, di-, and trihaloacetyl such as chloroacetyl, dichloroacetyl, trichloroacetyl, trifluoroacetyl; and carbonates including but not limited to alkyl carbonates having from one to six carbon atoms such as methyl, ethyl, n-propyl, isopropyl, n-butyl, t-butyl; isobutyl, and n-pentyl; alkyl carbonates having from one to six carbon atoms and substituted with one or more halogen atoms such as 2,2,2-trichloroethoxymethyl and 2,2,2-trichloroethyl; alkenyl carbonates having from two to six carbon atoms such as vinyl and allyl; cycloalkyl carbonates have from three to six carbon atoms such as cyclopropyl, cyclobutyl, cyclopentyl and cyclohexyl; and phenyl or benzyl carbonates optionally substituted on the ring with one or more C₁₋₆alkoxy, or nitro.

The present invention provides novel 10-desacetoxytaxol derivatives of the formula (1) 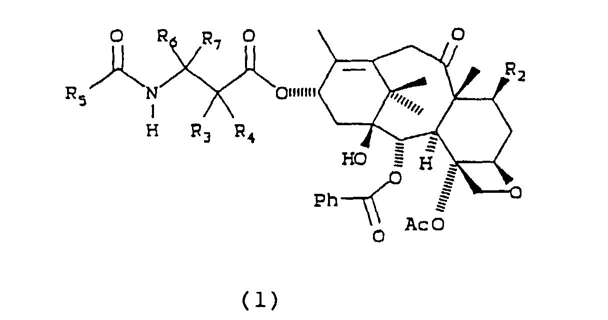 wherein R₂ is hydrogen, hydroxy or a protected hydroxy group; R₃ and R₄ are independently hydrogen, hydroxy, a protected hydroxy group, methyl, -SH, -NH₂, or -NR₈R₉; R₅ is R₁₀, or -OR₁₀; R₆ and R₇ are independently hydrogen, alkyl, or aryl; R₈ and R₉ are independently hydrogen, alkyl, alkenyl, alkynyl, or aryl; and R₁₀ is alkyl, alkenyl, alkynyl, or aryl; provided that when R₂ is hydrogen, R₃ and R₄ are independently hydrogen, methyl, -SH, -NH₂ or -NR₈R₉.

Exemplary compounds within the generic formula are depicted hereinbelow:

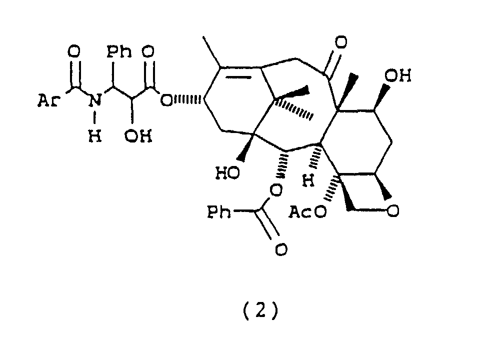

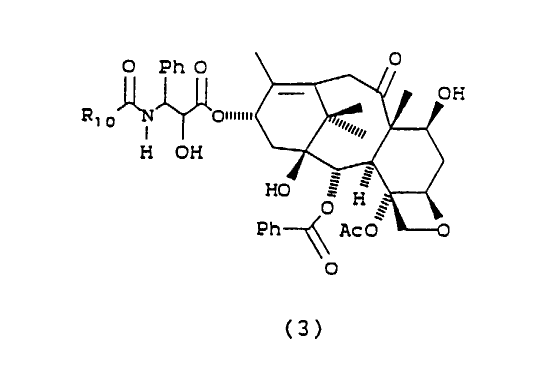

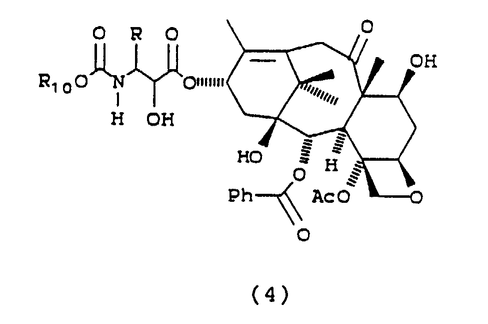

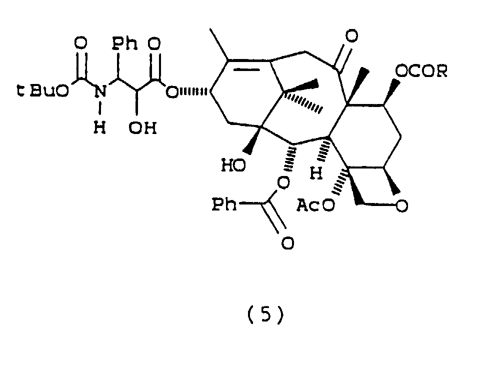

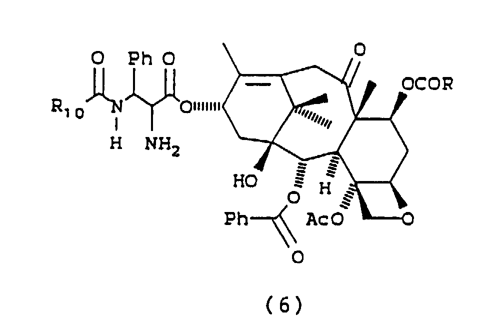

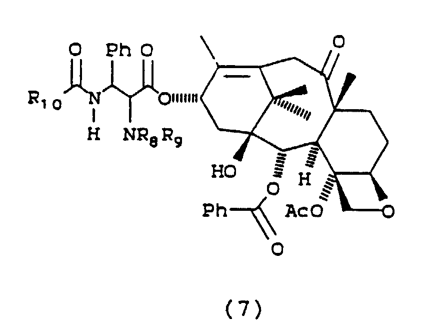

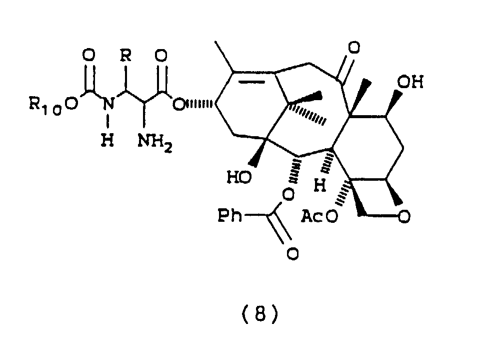

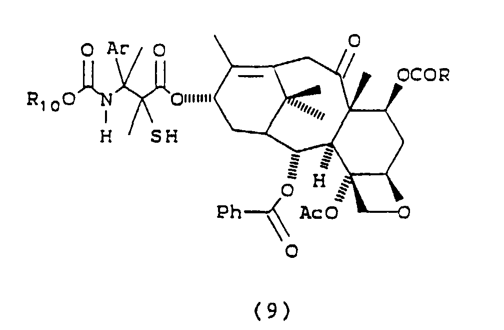

Preferably, one of R₃ and R₄ is hydrogen or methyl, while the other is hydroxy, or a protected hydroxy group. Preferably R₅ is phenyl, p-substituted phenyl or lower alkoxy, and most preferably phenyl, methoxy, ethoxy, or t-butoxy. Preferably, one of R₆ and R₇ is hydrogen or lower alkyl and the other is phenyl or naphthyl. Preferably R₈ is hydrogen or lower alkyl and R₉ is an amine protecting group.

In one preferred embodiment of compounds of formula (1), R₂ is hydroxy or a protected hydroxy group; R₃ is hydrogen; R₄ is hydroxy or a protected hydroxy group; R₅ is phenyl; R₆ is hydrogen and R₇ is phenyl. In another preferred embodiment, R₅ is t-butoxy. Where either R₃ or R₄ is a protected hydroxy group, it is preferably one of the carbonate type, most preferably it is selected from the group consisting of 2,2,2-trichloroethoxycarbonyl, allyloxycarbonyl, and benzyloxycarbonyl; or one of the trialkylsilyl type, most preferably triethylsilyl. The most preferred embodiments of compounds of formula (1) are: 1) 10-desacetoxytaxol; and 2) 10-deoxytaxotere.

A method for preparing compounds of formula (1) is provided by following the reaction sequence depicted in Scheme I.

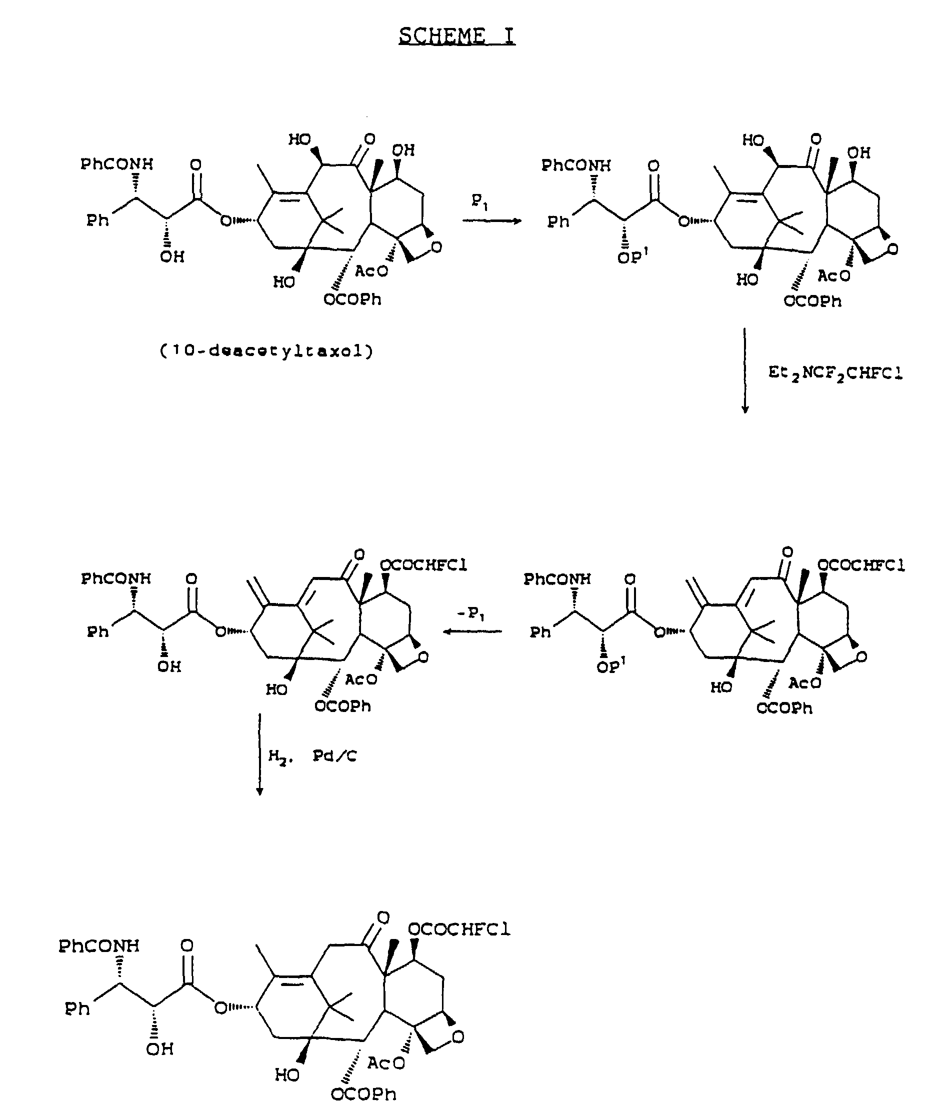

As illustrated, the starting materials used for the preparation of compounds of formula (1) using Scheme 1 include 10-deacetyltaxol, which may be obtained from taxol by treating the latter with zinc bromide in chloroform/ methanol as reported in G. Samaranayake et al., J. Org. Chem., 1991, 56:5114-5119, hereby incorporated by reference.

In Scheme I, P₁ is a hydroxy protecting group. Thus in the first reaction step, the 2'-hydroxy group of 10-deacetyltaxol is protected; the protecting group may be one that can be introduced and removed without unacceptably affecting the rest of the molecule, more preferably it may be one that preferentially blocks the 2'-hydroxy group over the two secondary hydroxy groups on the ring, namely, the 7- and 10-hydroxy groups. Protection of a hydroxy group may be accomplished by methods well known in the art; for example, reacting with a carboxylic acid or its acylating equivalent to form an ester, reacting with an alkyl halide in the presence of a base to form an ether, or reacting with a chloroformate to form the carbonate.

The preferred 2'-hydroxy protecting group in this reaction is a carbonate which can be formed by treating 10-deacetyltaxol with an appropriate chloroformate, for example, allyl or benzyl chloroformate to form the corresponding carbonate. The reaction is carried out in an inert organic solvent such as dichloromethane, tetrahydrofuran, acetonitrile, dimethylformamide, benzene, pyridine, p-dioxan, and preferably in the presence of an acid scavenger such as an amine base, for example, pyridine, diisopropylethylamine, 4-aminopyridine, triethylamine and the like, or an inorganic base such as potassium carbonate or tetrabutylammonium hydroxide, at a suitable temperature which may be -78°C to about 50°C, depending on the particular reagents chosen. Generally from about one to ten equivalents of the protecting group reagent relative to the taxol compound may be used; it is advantageous to monitor the reaction for example by thin-layer chromatography so as to achieve the desired degree of protection.

The 2'-hydroxy protected 10-deacetyltaxol thus obtained is then treated with 1,1,2-trifluoro-2-chlorotriethylamine (hereinafter TFCT) to give 2'-hydroxy protected 7-O-chlorofluoroacetyl-10-desacetoxy-11,12-dihydrotaxol-10,12(18)-diene. TFCT can be prepared from diethylamine and trifluorochloroethylene according to the method reported in N. N. Yarovenko, J. Org. Chem. USSR (English), 1959, 29:2125-8, hereby incorporated by reference. The reaction of 2'-hydroxy protected 10-deacetyltaxol and TFCT is carried out in an inert organic solvent such as halogenated hydrocarbons, for example, dichloromethane, chloroform, and carbon tetrachloride, at a temperature of from about 0°C up to the refluxing temperature of the reaction solution, preferably the reaction is run at ambient temperature. The TFCT is used in at least 1 equivalent to the taxol reactant, but preferably it is used in excess; typically from about one to about ten equivalents of TFCT may be used relative to the taxol reactant.

The 2'-hydroxy protecting group can be removed using methods known in the art that are suitable for the particular protecting group used; for example, acid or base catalyzed hydrolysis, reduction, hydrogenolysis, and the like. Thus the allyl carbonate can be removed by tributyltin hydride and tetrakis(triphenylphosphine)-palladium; the benzyl carbonate can be removed by catalytic hydrogenolysis.

7-O-Chlorofluoroacetyl-10-desacetoxy-11,12-dihydrotaxol-10,12(18)-diene can be converted to 10-desacetoxy-7-O-chlorofluoroacetyltaxol by catalytic hydrogenation in which the catalyst may be, for example, palladium, platinum, rhodium and the like. In cases where the 2'-hydroxy protecting group used in the previous steps of the reaction sequence is benzyloxycarbonyl, catalytic hydrogenation converts the 10,12-diene into the 11-ene, and removes the 2'- protecting group in one step. It is understood that the order of deprotecting the 2'-hydroxy group and hydrogenation is not crucial, and either may be effected prior to the other.

A further method for preparing compounds of the present invention provides compounds of formula (1) wherein R₂ is hydrogen, hydroxy, or protected hydroxy; and R₅ is phenyl. Compounds of this type may be prepared from 10-deacetyltaxol or 10-deacetyl-7-epitaxol as depicted in Scheme II.

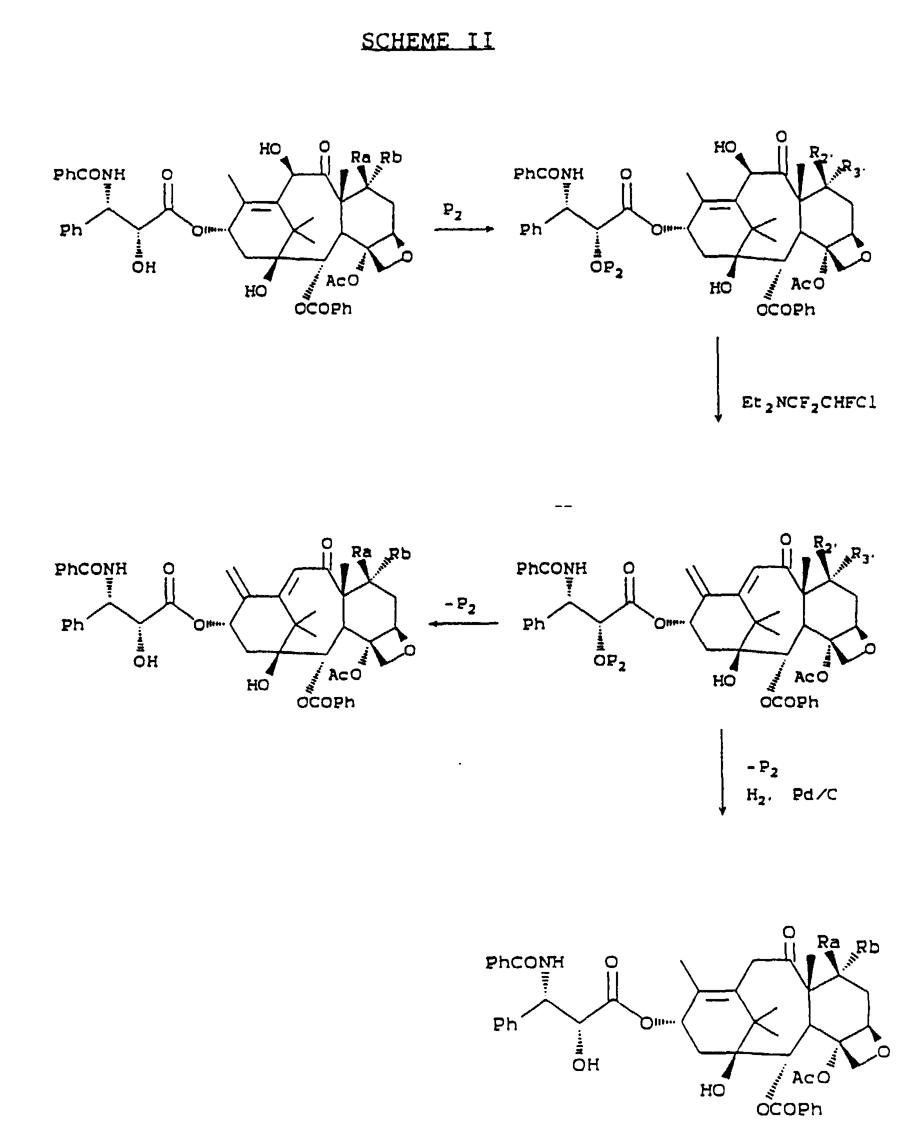

In Scheme II, Rₐ is hydroxy, R_{b} is hydrogen, R₂, is OP₂, and R₃, is hydrogen; or Rₐ is hydrogen, R_{b} is hydroxy, R₂, is hydrogen, and R₃, is hydroxy; P₂ is a hydroxy protecting group. When 10-deacetyltaxol (i.e. Rₐ is hydroxy and R_{b} is hydrogen) is the starting material, it is desirable to protect both the 2'- and the 7-hydroxy groups, and that the 10-hydroxy group be left free; preferably the protecting group is 2,2,2-trichloroethoxycarbonyl which can be introduced by reacting the taxol reactant with 2,2,2-trichloroethyl chloroformate in the presence of a base such as pyridine, diisopropylamine, triethylamine, dimethylaminopyridine, and the like. To control the degree of protection and to minimize blocking the 10-hydroxy group, the base is usually used in about one to two equivalents relative to taxol and the chloroformate in about 0.6 to about 1.5 equivalents relative to taxol. When 10-deacetyl-7-epitaxol (i.e. Rₐ is hydrogen and R_{b} is hydroxy) is the starting material, only the 2'-hydroxy group is protected since the 7-hydroxy group is substantially more inert than the 2'-hydroxy group, and is relatively insusceptible to acylation, either with a chloroformate, or with the TFCT in the subsequent step.

The 2',7-bishydroxy protected 10-deacetyltaxol or 2'-hydroxy protected l0-deacetyl-7-epitaxol thus obtained is then treated with TFCT to give 2',7-bishydroxy protected 10-desacetoxy-11,12-dihydrotaxol-10,12(18)-diene or 2'-protected 10-desacetoxy-11,12-dihydro-7-epitaxol-10,12(18)-diene, respectively. The reaction with TFCT is carried out in an inert organic solvent such as halogenated hydrocarbons such as dichloromethane, chloroform and carbon tetrachloride, at a temperature of from about 0°C to about the refluxing temperature of the reaction solution, preferably the reaction is run at ambient temperature. The TFCT is used in at least 1 equivalent to the taxol reactant, but preferably it is used in excess; typically TFCT is used from about one to about ten equivalents. The 2'- and, if present, the 7-hydroxy protecting group is then removed using a method suitable for the particular protecting group to provide 10-desacetoxy-11,12-dihydrotaxol-10,12(18)-diene or 10-desacetoxy-11,12-dihydro-7-epitaxol-10,12(18)-diene; for example, trichloroethyoxycarbonyl group is removed by a zinc reagent, benzyloxycarbonyl is removed by hydrogenolysis, and allyl carbonate is removed by tributyltin hydride and tetrakis(triphenylphosphine)palladium. 10-Desacetoxy-11,12-dihydrotaxol-10,12(18)-diene and 10-desacetoxy-11,12-didehydro-7-epitaxol-10,12(18)-diene may then be subjected to catalytic hydrogenation as previously discussed to provide 10-desacetoxytaxol and 10-desacetoxy-7-epitaxol, respectively.

2',7-Bishydroxy protected 10-desacetoxy-11,12-dihydrotaxol-10,12(18)-diene or 2'-hydroxy protected 10-desacetoxy-11,12-dihydro-7-epitaxol-10,12(18)-diene can also be subjected to catalytic hydrogenation to give 2',7-bishydroxy protected 10-desacetoxytaxol or 2'-hydroxy protected 10-desacetoxy-7-epitaxol, respectively. The hydroxy protecting groups may be removed as previously described to provide 10-desacetoxytaxol or 10-desacetoxy-7-epitaxol.

A further method for preparing compounds of the present invention provides compounds of formula (1) wherein R₂ is hydroxy or a protected hydroxy group, R₃ is hydrogen; R₄ is hydrogen, hydroxy, or a protected hydroxy group; R₅ is t-butoxy; R₆ is hydrogen and R₇ is phenyl. Compounds of this type may be prepared from 7-protected 10-desacetoxy baccatin III or 10-desacetoxy-7-epi-baccatin III using the process shown in Scheme III.

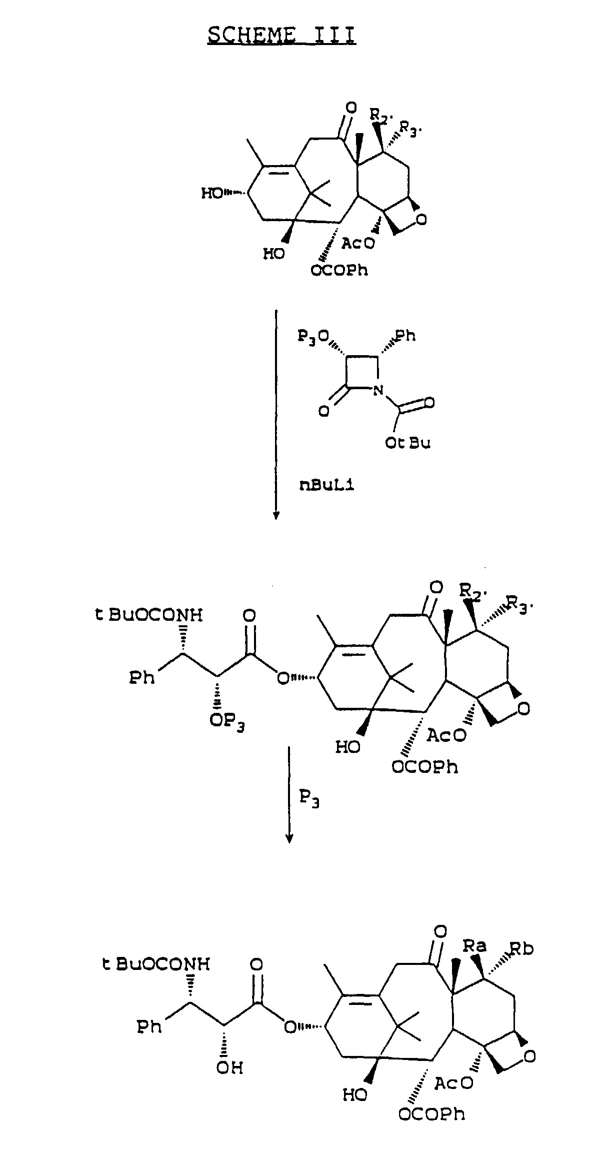

In Scheme III, Rₐ is hydroxy, R_{b} is hydrogen, R₂, is -OP₃, and R₃, is hydrogen; or Rₐ is hydrogen, R_{b} is hydroxy, R₂, is hydrogen, and R₃, is hydroxy; P₃ is a hydroxy protecting group; preferably it is a trialkylsilyl group, most preferably triethylsilyl. 7-Hydroxy protected 10-desacetoxy baccatin III or 10-desacetoxy-7-epi-baccatin III is treated with a strong base such as n-butyl lithium to generate the corresponding 13-alkoxide. The alkoxide is reacted with (3R,4S)-1-t-butoxycarbonyl-4-phenyl-3-protected hydroxy-2-azetidinone in an inert organic solvent such as tetrahydrofuran to provide the corresponding 2',7-bishydroxy protected 10-deoxytaxotere. The reaction is carried out at a low temperature in the range of about -20°C to about 25°C, preferably at about 0°C. The hydroxy protecting groups are then removed to provide 10-deoxytaxotere using conventional methods, for example triethylsilyl group may be removed by acid hydrolysis with hydrochloric acid.

The reaction sequence depicted in Scheme III shows the preparation of 10-deoxytaxotere or 10-deoxy-7-epitaxotere; however, it is equally applicable for preparing 10-desacetoxytaxol or 10-desacetoxy-7-epitaxol. Thus, 10-desacetoxytaxol and 10-desacetoxy-7-epitaxol may be obtained in an analogous manner by replacing the β-lactam shown in Scheme III with (3R,4S)-1-benzoyl-4-phenyl-3-hydroxy protected-2-azetidinone.

Other derivatives of 10-desacetoxytaxol within the scope of formula (1) may readily be prepared by selection of the proper substituents for the β-lactam which forms the β-amido ester side chain at C-13 of the taxol structure.

10-Desacetoxybaccatin III and 10-desacetoxy-7-epibaccatin III may be obtained from 10-deacetylbaccatin III and 10-deacetyl-7-epibaccatin III by the process shown in Scheme IV.

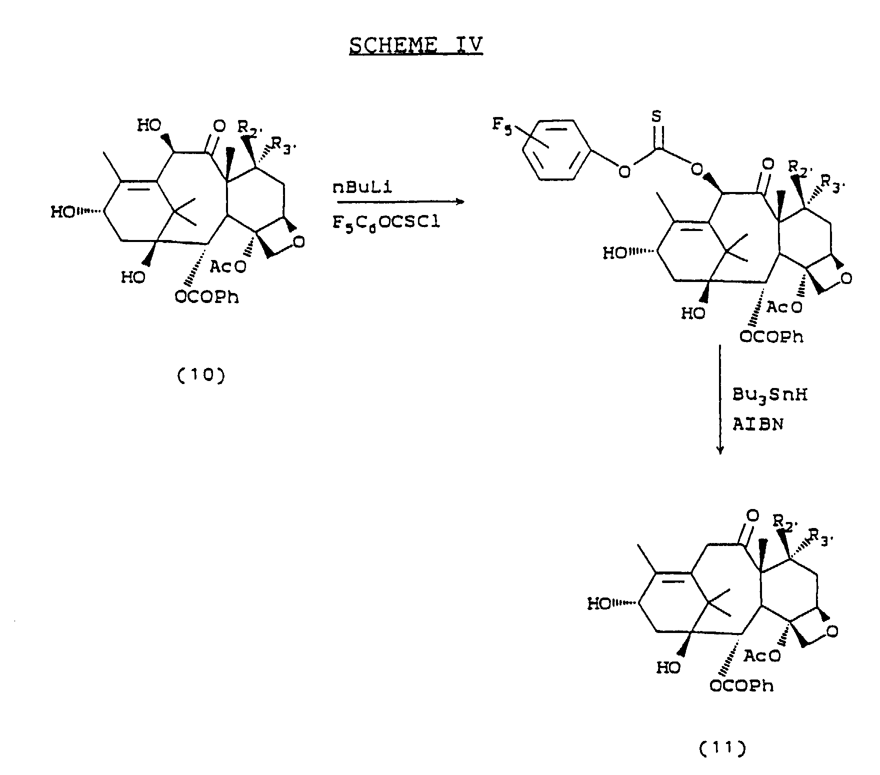

In Scheme IV, R₂, is hydrogen and R₃, is hydroxy, or R₂, is protected hydroxy and R₃, is hydrogen. Preferably, the hydroxy protecting group is trialkylsilyl ether type, for example, triethylsilyl. Thus 10-deacetylbaccatin III is first treated with a strong base such as n-butyl lithium, and alkoxide generated in situ is reacted with pentafluorophenyl chlorothionoformate to provide the corresponding l0-(pentafluorophenyl)thiocarbonate. The reaction is carried out in an inert organic solvent such as tetrahydrofuran, at a temperature in the range of about -78°C to about room temperature. The thiocarbonate thus obtained is treated with a suitable reagent such as tributyltin hydride, triphenyltin hydride, trimethylsilane, and tributyltinchloride and sodium cyanoborohydride, in the presence of a radical initiator such as azobisisobutyronitrile (AIBN). The reaction is carried out in an organic solvent such as t-butanol, N-methylpyrrolidone, dimethylsulfoxide, dimethylformamide, toluene and benzene at a temperature from about 80°C to about 115°C to provide 7-protected hydroxy-10-desacetoxy baccatin III or 7-epibaccatin III. Alternatively, the reaction can be carried out under photochemical conditions (triorganotin hydride, hv, benzene, 0°C - room temperature). The hydroxy protecting group may be removed using methods known in the art; for example, triethylsilyl can be removed by acid hydrolysis using hydrochloric acid.

The 10-desacetoxytaxol derivatives of the present invention are useful agents for inhibiting tumor growth in animals and human. Compounds were evaluated in in vitro cytoxicity activity against human colon carcinoma cells HCT-116 and HCT-116/VM46. The HCT116/VM cells are cells that have been selected for teniposide resistance and express the multidrug resistance phenotype, including resistance to taxol. Cytotoxicity was assessed in HCT-116 human colon carcinoma cells by XTT (2,3-bis(2-methoxy-4-nitro-5-sulfophenyl)-5-[(phenylamino)carbonyl]2H-tetrazolium hydroxide) assay as reported in D.A. Scudiero, et al., "Evaluation of soluble tetrazolium/formazan assay for cell growth and drug sensitivity in culture using human and other tumor cell lines," Cancer Res. 48:4827-4833, 1988. Cells were plated at 4000 cells/well in 96 well microtiter plates and 24 hours later drugs were added and serial diluted. The cells were incubated at 37°C for 72 hours at which time the tetrazolium dye, XTT, was added. A dehydrogenase enzyme in live cells reduces the XTT to a form that absorbs light at 450 nm which can be quantitated spectrophotometrically. The greater the absorbance, the greater the number of live cells. The results are expressed as an IC₅₀, which is the drug concentration required to inhibit cell proliferation (i.e., absorbance at 450 nm) to 50% of that of untreated control cells. The IC₅₀ values for 10-desacetoxytaxol and taxol are given in Table I.

**TABLE I**

| | | |
|---|---|---|
| In vitro cytotoxicity for taxol analogs against human colon carcinoma cells. | | |

| | **IC**_{**50**} **(µM)** | |
|---|---|---|
| **Compound** | **HCT-116** | **HCT-116/VM46** |
| 10-Desacetoxytaxol | 0.008 | 1.22 (153) |
| Taxol | 0.004 | 0.440 (124) |
| * Value in parenthesis is fold resistance relative to HCT-116 cells. | | |

Compounds of formula (1) of the instant invention are useful for inhibiting tumor growth in animals including humans and are preferably administered in the form of a pharmaceutical composition comprising an effective antitumor amount of compound of the instant invention in combination with a pharmaceutically acceptable carrier or diluent.

Antitumor compositions herein may be made up in any suitable form appropriate for desired use; e.g., oral, parenteral or topical administration. Examples of parenteral administration are intramuscular, intravenous, intraperitoneal, rectal and subcutaneous administration.

The diluent or carrier ingredients should not be such as to diminish the therapeutic effects of the antitumor compounds.

Suitable dosage forms for oral use include tablets, dispersible powders, granules, capsules, suspensions, syrups, and elixirs. Inert diluents and carriers for tablets include, for example, calcium carbonate, sodium carbonate, lactose and talc. Tablets may also contain granulating and disintegrating agents such as starch and alginic acid, binding agents such as starch, gelatin and acacia, and lubricating agents such as magnesium stearate, stearic acid and talc. Tablets may be uncoated or may be coated by unknown techniques; e.g., to delay disintegration and absorption. Inert diluents and carriers which may be used in capsules include, for example, calcium carbonate, calcium phosphate and kaolin. Suspensions, syrups and elixirs may contain conventional excipients, for example, methyl cellulose, tragacanth, sodium alginate; wetting agents, such as lecithin and polyoxyethylene stearate; and preservatives, e.g., ethyl-p-hydroxybenzoate.

Dosage forms suitable for parenteral administration include solutions, suspensions, dispersions, emulsions and the like. They may also be manufactured in the form of sterile solid compositions which can be dissolved or suspended in sterile injectable medium immediately before use. They may contain suspending or dispersing agents known in the art.

One aspect of the invention herein is directed to therapeutically inhibiting tumor growth in an animal host having a tumor sensitive to the compounds of the instant invention which comprises administering to said host an effective antitumor dose of said compound. It will be appreciated that the actual preferred amount of compound of the instant invention will vary according to the particular compound, the particular composition formulated, the mode of application and the particular situs, host and disease being treated. Many factors that modify the action will be taken into account by those skilled in the art; e.g., age, body weight, sex, diet, time of administration, route of administration, rate of excretion, condition of the host, drug combinations, reaction sensitivities and severities and severity of disease. Administration can be carried out continuously or periodically within the maximum tolerated dose. Optimal application rates for a given set of conditions can be ascertained by those skilled in the art using conventional dosage administration tests in view of the above guidelines.

The following examples are provided in order to more fully illustrate the present invention.

### EXAMPLE 1

### 2',7-bis'O-(2,2,2-trichloroethoxycarbonyl)-10-deacetyl taxol (12).

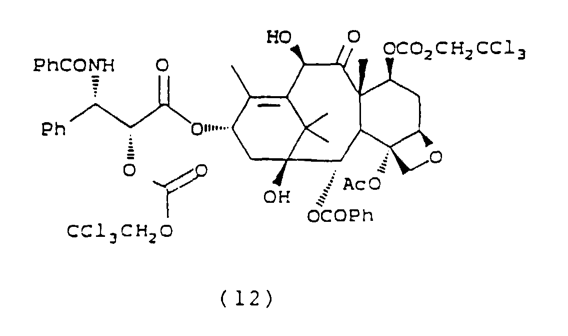

10-Deacetyl taxol (140 mg, 0.173 mmol) in dry dichloromethane (3.5 mL) was treated at O°C with pyridine (0.028 mL, 0.346 mmol) and trichloroethyl chloroformate (0.0724 mL, 0.260 mmol). After lh at this temperature, the cold bath was removed and the mixture was stirred at room temperature overnight. The solvent was evaporated and the residue chromatographed on silica gel (30-50% ethyl acetate in hexane) to afford 12 as a foam (92.3 mg, 46%). Further elution afforded unreacted starting material (35 mg, 25%). Carbonate 12 had NMR (300 MHz, CDCl₃) δ 8.14 (d, J=8.5 Hz, 2H) 7.75 (d, J=8.5 Hz, 2H) 7.65-7.35 (m, 11H) 6.94 (exch.d, J=9.3 Hz, 1H) 6.27 (br t, 1H) 6.04 (dd, J=9.3 Hz, J'=2.6 Hz, 1H) 5.71 (d, J=6.9 Hz, 1H) 5.54 (d, J=2.6 Hz, 1H) 5.43-5.37 (m, 2H) 4.96 (d, J=7.9 Hz, 1H) 4.85-4.67 (m, 4H) 4.29 (AB q, 2H) 4.04-4.01 (m, 2H) 2.69- 1.80 (m, 14H incl. singlets at 2.58, 1.96, 1.89, 3H each) 1.20 (s, 3H) 1.09 (s, 3H).
High Res. Mass Spectrum: Calcd for C₅₁H₅₁NO₁₇Cl₆K (MK+) 1198.0925, found 1198.0949.

### EXAMPLE 2

### 2',7-bis'O-(2,2,2-trichloroethoxycarbonyl)-10-desacetoxy-11,12-dihydrotaxol-10,12(18)-diene (13).

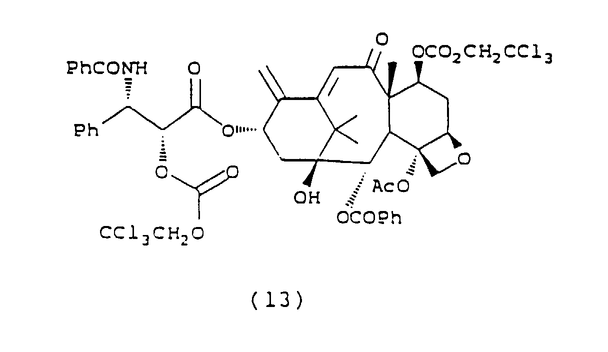

Bis-carbonate 12 (92.3 mg, 0.079 mmol) in dry dichloromethane (2 mL) was treated at room temperature with TFCT (0.0384 mL, 0.238 mmol). The solution was stirred overnight. The solvent was evaporated and the residue purified by column chromatography (25% ethyl acetate in hexane) to afford 13 as a white powder (42.8 mg, 47.3%). NMR (300 MHz, CDCl3) δ 8.18 (d, J=8.5 Hz, 2H) 7.69 (d, J=8.5 Hz, 2H) 7.58-7.28 (m, 11H) 6.90 (exch. d, J=9.7 Hz, 1H) 6.31 (s, 1H) 6.25 (br t, 1H) 6.04 (dd, J=9.7 Hz, J'=2.2 Hz, 1H) 5.80 (d, J=7.8 Hz, 1H) 5.51 (d, J=2.2 Hz, 1H) 5.38 (d, J=2.3 Hz, 1H) 5.31 (dd, J= 11.0 Hz, J'=7.3 Hz, 1H) 4.99 (d, J=2.3 Hz, 1H) 4.97 (br d, 1H) 4.80-4.65 (m, 4H) 4.30 (AB q, 2H) 3.75 (d, J=7.8 Hz, 1H) 2.60 (m, 1H) 2.55 (s, 3H) 2.16-2.09 (m, 3H) 1.83 (s, 3H) 1.77 (s, 1H) 1.13 (s, 3H) 1.07 (s, 3H).
High Res. Mass Spectrum: Calcd for C₅₁H₄₉NO₁₆Cl₆K (MK+) 1180.0820, found 1180.0777.

### EXAMPLE 3

### 10-Desacetoxy-11,12-dihydrotaxol-10,12(18)-diene (14).

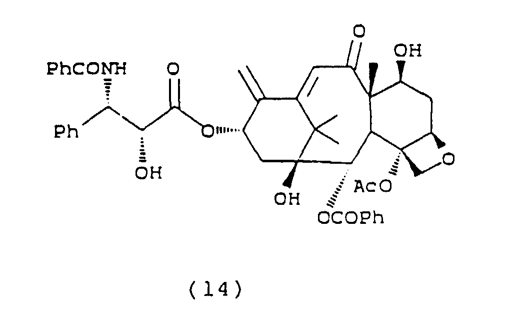

Dienone 13 (180 mg, 0.157 mmol) was dissolved in methanol (3 mL) and treated with acid-washed Zn dust (300 mg, 4.72 mmol). The slurry was refluxed for 20 min, filtered, and the filtrate evaporated. Chromatography of the residue (40-60% ethyl acetate in hexane) gave 14 as a foam (18 mg, 14%), together with its 7-epi isomer (97 mg, 77.7%). Compound 14 had NMR (300 MHz, CDCl₃) δ 8.19 (d, J=8.5 Hz, 2H) 7.69 (d, J=8.5 Hz, 2H) 7.59-7.25 (m, 11H) 7.00 (exch. d, J=10.4 Hz, 1H) 6.20 (br t, 1H) 5.96 (s, 1H) 5.83-5.77 (m, 2H) 5.18 (d, J-2.2 Hz, 1H) 4.94 (dd, J=7.2 Hz, J'=2.1 Hz, 1H) 4.79 (d, J-2.2 Hz, 1H) 4.72 (dd, J-4.3 Hz, J'=2.2 Hz, 1H) 4.30 (AB q, 2H) 4.00 (m, 1H) 3.65 (d, J=7.8 Hz, 1H) 3.51 (exch. d, J=4.3 Hz, 1H) 2.60 (m, 1H) 2.40 (s, 3H) 2.30-1.80 (m, 5H) 1.70 (s, 3H) 1.16 (s, 3H) 1.06 (s, 3H). High Res. Mass Spectrum: Calcd for C₄₅H₄₈NO₁₂ (MH+) 794.3177, found 794.3152.

Alternatively, dienone 13 (39 mg, 0.034 mmol) was dissolved in methanol (0.5 mL) and acetic acid (0.5 mL), and treated with acid-washed zinc dust (66.4 mg, 1.020 mmol). The slurry was heated at 40°C for 1h, filtered and the filtrate evaporated. Chromatography of the residue with 60% ethyl acetate/hexane gave 14 as a foam (22 mg, 81%). Spectral data are the same as the previous ones.

### EXAMPLE 4

### 10-Desacetoxytaxol (15).

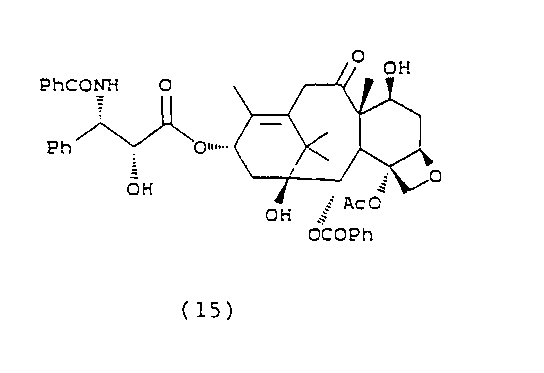

Dienone 14 (22 mg, 0.028 mmol) in ethyl acetate (0.7 mL) was hydrogenated at atmospheric pressure in the presence of palladium on charcoal (10%, 14.7 mg, 0.014 mmol Pd) After 5.5 h at RT, filtration (rinsing with ethyl acetate), evaporation and chromatography (60% ethyl acetate in hexane) gave 10-deoxytaxol 15 (15.0 mg, 68%) as a white foam. NMR (CDCl₃) δ 8.12(d, J=8.5 Hz, 2H) 7.61 (d, J=8.4 Hz, 2H) 7.60-7.27 (m, 11H) 7.06 (exch. d, J=8.9 Hz, 1H) 6.09 (br t, 1H) 5.78 (dd, J= 3.9 Hz, J'=2.6 Hz, 1H) 5.66 (d, J=6.8 Hz, 1H) 4.91 (d, J=7.6 Hz, 1H) 4.76 (dd, J= 5.1 Hz, J'= 2.6 Hz, 1H) 4.29-4.20 (m, 3H) 4.01 (d, J=6.8 Hz, 1H) 3.75 (d, J=15.8 Hz, 1H) 3.60 (exch. d, J=5.1 Hz, 1H) 3.39 (br d, 1H) 2.60 (m, 1H) 2.34 (s, 3H) 2.34-2.22 (m, 2H) 1.90- 1.71 (m, 2H) 1.62 (s, 3H) 1.61 (s, 3H) 1.53 (exch. d, J=7.8 Hz, 1H) 1.14 (s, 3H) 1.12 (s, 3H).
High Res. Mass Spectrum: Calcd for C₄₅H₅₀NO₁₂ (MH+) 796.3333, found 796.3319.

### EXAMPLE 5

### 10-Desacetoxy-7-O-triethylsilyl baccatin III (16).

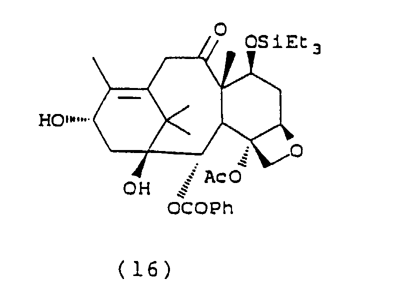

### A. 10-desacetyl-7-O-triethylsilyl baccatin III (Compound A).

10-Desacetyl baccatin III (from Taxus baccata, 628.0 mg, 1.150 mmol) was dissolved in dry DMF (6 mL), cooled to 0°C, and treated with imidazole ( 312.8 mg, 4.595 mmol) and chlorotriethylsilane (0.772 mL, 4.60 mmol). The mixture was stirred at 0°C for 4 h, then diluted with ethyl acetate ( 150 mL) and washed exhaustively with water and brine. The organic layer was dried and concentrated. The residue was chromatographed (silica, 50% ethyl acetate in hexane), to afford compound A as a foam (586 mg, 77%). This compound is described by Greene et al. in J. Am. Chem. Soc., 1988, 110, 5917.

### B. 10-desacetyl-7-O-triethylsilyl-10-Q-(pentafluorophenyloxy)thiocarbonyl baccatin III (Compound B).

Compound A (319 mg, 0.485 mmol) was dissolved in dry THF (5 mL), cooled to -40°C, and treated with n-butyllithium (1.58M in hexanes, 0.384 mL, 0.606 mmol). After 40 min at this temperature, pentafluorophenyl chlorothionoformate (0.086 mL, 0.536 mmol) was added neat by syringe. The reaction mixture was stirred at -20°C for 90 min, then quenched with saturated ammonium chloride solution, and extracted with ethyl acetate. The ethyl acetate layer was dried, evaporated and the residue chromatographed (silica, 40% ethyl acetate in hexane) to afford Compound B as a foam (320 mg, 74%). NMR (CDCl₃) δ 8.09 (d, 2H) 7.56 (t, 1H) 7.44 (m, 2H) 6.78 (s, 1H) 5.64 (d, J-6.9 Hz, 1H) 4.96-4.89 (m, 2H) 4.49 (dd, J=10.2 Hz, J'=6.6 Hz, 1H) 4.12 (AB q, 2H) 3.80 (d, J=6.9 Hz, 1H) 2.55-0.44 (m, 43H).
Mass Spectrum: 884 (MH+).

### C. 10-desacetoxy-7-O-triethylsilyl baccatin III.

Thionocarbonate (compound B, 119 mg, 0.135 mmol) was dissolved in dry toluene (3 mL) and treated with 2,2'-azobisisobutyronitrile (AIBN, 2 mg). The solution was degassed with dry nitrogen, then tributyltin hydride (0.055 mL, 0.202 mmol) was added and the solution was heated for 1 h (90°C). Solvent evaporation and chromatography (silica, 40% ethyl acetate in hexane) gave the title product (87 mg, 99%) as a colorless foam. NMR (CDCl₃) δ 8.07 (d, J=8.2 Hz, 2H) 7.56 (br t, 1H) 7.44 (m, 2H) 5.57 (d, J=6.7 Hz, 1H) 4.92 (d, J=9.3 Hz, 1H) 4.78 (br s, 1H) 4.48 (dd, J=10.4 Hz, J'=6.6 Hz, 1H) 4.09 (AB q, 2H) 4.06 (d, J=6.7 Hz, 1H) 3.74 (d, J=14.8 Hz, 1H) 3.35 (br d, 1H) 2.44 (m, 1H) 2.25 (s, 3H) 2.22-0.45 (m,42H).
Mass spectrum: 642 (MH+).

### EXAMPLE 6

### 10-Deoxytaxotere (17).

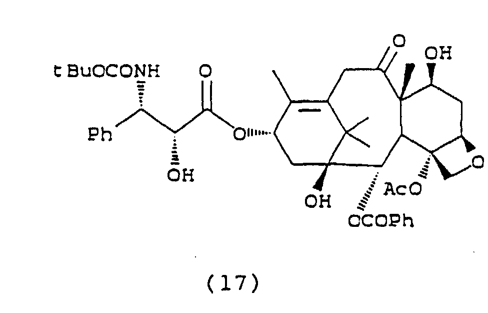

Compound 16 (100 mg, 0.156 mmol) was placed in a flask under Argon and dissolved in dry THF (1.5 mL). Upon cooling to -40°C, n-butyllithium (1.45M in hexanes, 0.119 mL, 0.170 mmol) was added dropwise, followed by (3R,4S)-1-tert-butoxycarbonyl-4-phenyl-3-triethylsilyloxy-2-azetidinone 94.2 mg, 0.25 mmol) in THF (0.5 mL) over a period of 2 min. The mixture was immediately warmed to 0°C and stirred for 45 min before quenching with saturated ammonium chloride (3 mL). The mixture was extracted with ethyl acetate, dried, and evaporated. Silica gel chromatography (30% ethyl acetate in hexane ) afforded 2',-7-bis-O-(triethylsilyl)-10-deoxytaxotere as a foam (125 mg. 76%). This compound (100mg, 0.098 mmol) was immediately dissolved in acetonitrile (2 mL) at -5°C and treated with hydrochloric acid (0.037 mL, 36%, 12M). The mixture was stirred for 2h at -5°C, then it was quenched with aqueous bicarbonate, extracted with ethyl acetate, and dried. Evaporation was followed by silica chromatography (75% ethyl acetate in hexane) to afford the title compound as a foam (80.5mg, 80%). NMR (CDCl₃) δ 8.10 (d, J=8.2 Hz, 2H) 7.64-7.29 (m, 8H) 6.11 (br t, 1H) 5.68 (d, J-6.9 Hz, 1H) 5.43 (br d, 1H) 5.25 (br d, 1H) 4.93 (d, J=7.7 Hz, 1H) 4.60 (br s, 1H) 4.30-4.18 (m, 3H) 4.02 (d, J=7.7 Hz, 1H) 3.80 (d, J=15.8 Hz, 1H) 3.46-3.40 (m, 2H) 2.62 (m, 1H) 2.35 (s, 3H) 2.35-2.25 (m, 2H) 1.89-1.65 (m, 5H) 1.63 (s, 3H) 1.35 (s, 9H) 1.19 (s, 3H) 1.16 (s, 3H).

### EXAMPLE 7

### Preparation of (3R,4S)-1-tertbutoxycarbonyl-4-phenyl-3-triethylsilyloxy-2-azetidinone (see Scheme V).

(L)-Threonine methyl ester hydrochloride (1.26 g, 7.44 mmol) in anhydrous dichloromethane (15 mL) was stirred with imidazole (1.01 g, 14.89 mmol) and t-butoxydiphenylsilyl chloride (2.274 g, 7.816 mmol) for 16 h at room temperature. The reaction mixture was partitioned between water and dichloromethane. The organic phase was washed with 5% aqueous sodium bicarbonate and water, dried and concentrated to give 2.88 g of a crude oil, which was used directly in the next step; 1H-NMR (CDCl₃) δ: 7.70-7.25 (m, 10H) 4.44 (m,lH) 3.62 (s, 3H) 3.31 (d, J=3 Hz, 1H) 2.12 (bs, 2H) 1.3-1.15 (m, 12H).

This oil (548 mg, 1.414 mmol) in anhydrous dichloromethane (10 mL) was treated with benzaldehyde (0.158 mL, 1.55 mmol) at rt overnight in the presence of 4Å molecular sieves to afford compound of formula XVIIa in situ. Upon cooling the solution containing compound XVIIa to -40°C, triethylamine (0.20 mL, 1.698 mmol) was added, followed by acetoxyacetyl chloride (XVIa) (0.182 mL, 1.698 mmol) over 10 min. The mixture was allowed to reach rt over 4 h and the product was partitioned between dichloromethane and water. The organic phase was further washed with water and brine, dried and concentrated. Silica gel chromatography (being eluted with 1:4 EtOAc/hexane) gave 411 mg of compound XVIIIa as a ca. 10:1 mixture of 3R,4S : 3S,4R diastereomers.

This mixture of diastereomers (245.1 mg, 0.414 mmol) in dry THF (2 mL) was treated with acetic acid (0.15 mL) and tetrabutylammonium fluoride (TBAF, 1M in THF, 1.20 mL). The solution was stirred for 14 h at rt, then partitioned between ethyl acetate and 5% aqueous sodium bicarbonate. The organic phase was dried and concentrated. Flash silica gel chromatography using 1:1 ethyl acetate : hexane as eluant gave 66 mg (Y: 50%) of compound XIXa as a foam in one diastereomer; 1H-NMR (CDCl₃) δ: 7.42-7.25 (m, 5H) 5.90 (d, J=4.8 Hz, 1H) 5.09 (d, J=4.8 Hz, 1H) 4.28 (m, 1H) 4.01 (d, J=4.8 Hz, 1H) 3.70 (s, 3H) 1.73 (s, 3H) 1.19 (d, J=6.6 Hz, 3H).

Compound of formula XIXa (9.8 g, 0.0305 mol) in dry dichloromethane (100 mL) was treated at -78°C with triethylamine (9.40 mL, 0.0671 mol) and methanesulfonyl chloride (MsCl, 3.50 mL, 0.0457 mol). The solution was allowed to reach rt overnight. The reaction mixture was partitioned between water and dichloromethane. The organic layer was washed with 5% aqueous sodium bicarbonate, dilute aqueous HCl, water and brine, and concentrated to afford compound XXa as a crude oily residue. The crude residue (10.0 g) was dissolved in dichloromethane (250 mL) and ozonized at -78°C until the color of the solution stayed as blue. Addition of methyl sulfide (11 mL) and concentration of the reaction mixture gave compound of formula XXIa (crude).

The compound of formula XXIa was dissolved in THF (150 mL) and treated at -78°C with hydrazine hydrate (10 mL). After 2 h, the mixture was poured into dilute aqueous HCl and ethyl acetate, and the two phases were separated. The organic phase was washed with more acid, water and brine and concentrated to afford a crude product, which was purified by silica gel chromatography using 1-5% methanol in methylene chloride to yield 4.40 g (Y: 71%) of compound of formula XXIIa; 1H-NMR (CDCl₃) δ: 7.38-7.24 (m, 5H) 6.31 (bs, 1H) 5.87 (bm, 1H) 5.04 (d, J=4.8 Hz, 1H) 1.67 (s, 3H).

To a cooled (-5°C) mixture of 1M aqueous KOH (140 mL) and acetonitrile (100 mL), a solution of compound XXIIa (2.39 g, 11.22 mmol) in acetonitrile (130 mL) was added dropwise. The mixture was stirred at 0°C for 1 h and diluted with ethyl acetate (300 mL), water (50 mL) and saturated aqueous bicarbonate (50 mL). The organic phase was separated, and the aqueous layer further extracted with ethyl acetate (3x200 mL). The organic phases were combined, dried, filtered and concentrated to give compound of formula XXIIIa (crude), which was recrystallized from hexane/acetone (mp, 184-6°C); yield, 1.53 g (Y: 82%).

To azetidinone XXIIIa (580 mg, 3.55 mmol) in dry THF (5.0 mL) was added imidazole (265.5 mg, 3.90 mmol), followed by triethylsilyl chloride (0.654 mL, 3.90 mmol). The mixture was allowed to be stirred for 1 h. Ethyl acetate was added and the organic layer was washed with brine, 10% aqueous HCl and dried. Silica gel chromatography (being eluted with 25% ethyl acetate in hexane) gave 670 mg (Y: 68%) of compound XXIVa as a foam.

To a stirred solution of compound XXIVa (2.20 g, 7.92 mmol) in dry THF (25 mL) was added ethyl diisopropylamine (1.65 mL, 9.51 mmol) at 0°C under argon atmosphere. The solution was stirred for 5 min, then di-tert-butyl carbonate (2.08 g, 9.51 mmol) and 4-dimethylaminopyridine (193.6 mg, 1.58 mmol) were added. The reaction mixture was stirred at 0°C for 60 min. The reaction was diluted with ethyl acetate (25 mL), and the mixture was washed with brine, 10% aqueous sodium bicarbonate, 10% aqueous HCl, dried over magnesium sulfate, and concentrated to leave an oil. Silica gel flash chromatography (being eluted with 15% ethyl acetate in hexane) gave 2.40 g (Y: 83%) of compound XVa as a white solid; 1H-NMR (CDCl₃) δ 7.28 (m, 5H) 5.03 (m, 2H) 1.38 (s, 9H) 0.76 (t, J = 7.56, 9H) 0.43 (m, 6H).

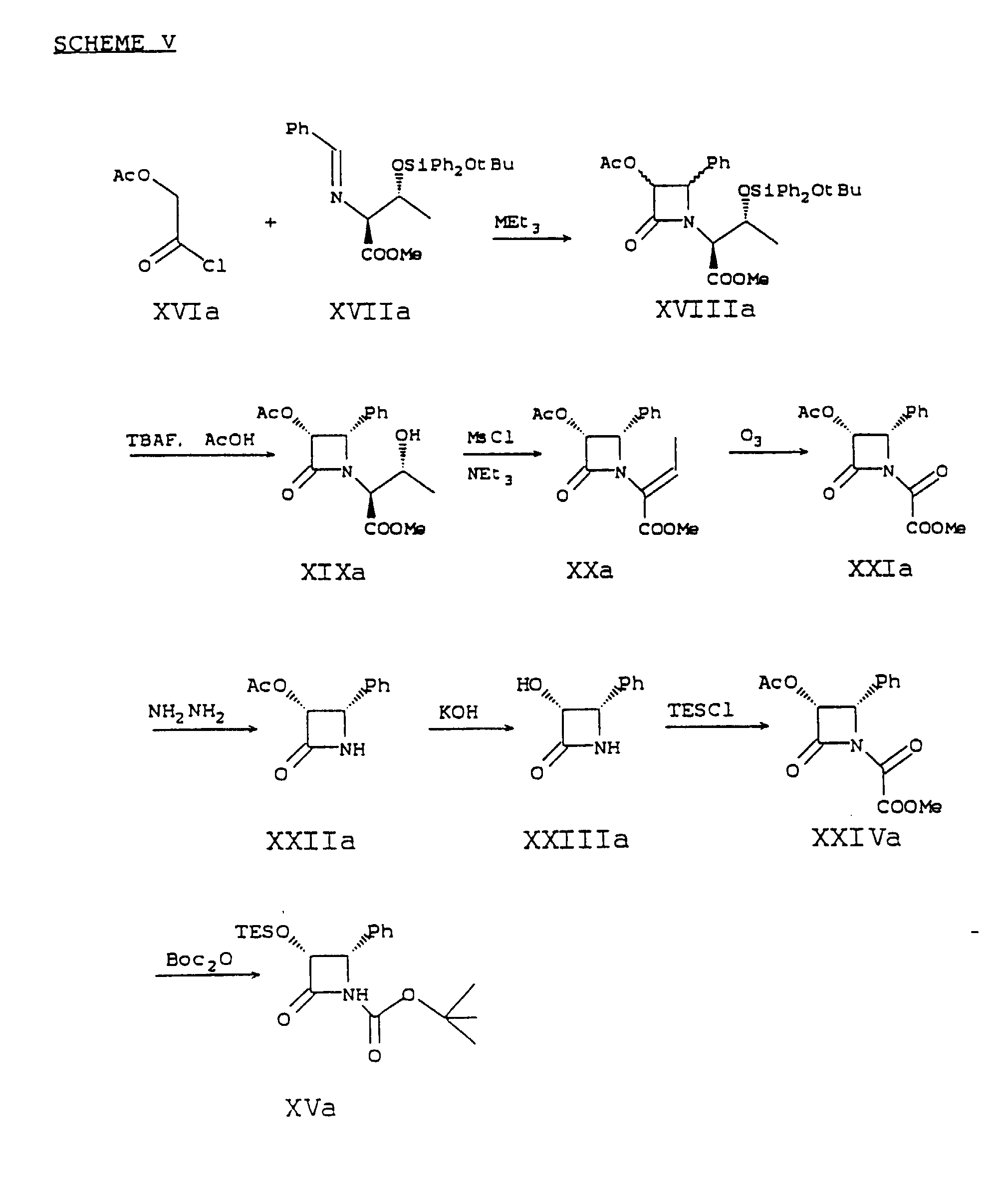

In view of the above, it will be seen that the several objects of the invention are achieved.

As various changes could be made in the above compositions and processes without departing from the scope of the invention, it is intended that all matter contained in the above description be interpreted as illustrative and not in a limiting sense.

## Claims (Claims for the following Contracting State(s): AT, BE, CH, LI, DE, DK, FR, GB, IE, IT, LU, MC, NL, SE)

1. A compound of the formula 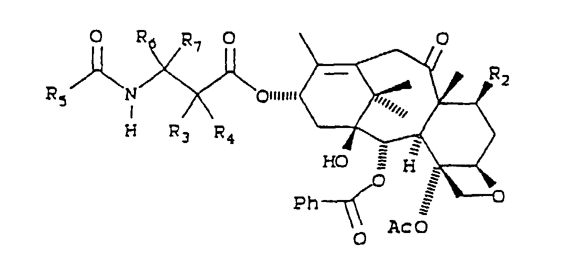 wherein R₂ is hydrogen, hydroxy or a protected hydroxy group; R₃ and R₄ are independently hydrogen, hydroxy, a protected hydroxy group, methyl, -SH, -NH₂, or -NR₈R₉; R₅ is R₁₀, or -OR₁₀; R₆ and R₇ are independently hydrogen, alkyl, or aryl; R₈ and R₉ are independently hydrogen, alkyl, alkenyl, alkynyl, or aryl; and R₁₀ is, alkyl, alkenyl, alkynyl, or aryl; provided that when R₂ is hydrogen, R₃ and R₄ are independently hydrogen, methyl, -SH, -NH₂, or -NR₈R₉.

2. A compound according to claim 1, wherein R₃ is hydrogen and R₄ is hydroxy or a protected hydroxy group, R₅ is phenyl or t-butoxy, R₆ is hydrogen, and R₇ is phenyl.

3. A compound according to claim 1, wherein R₃ is hydrogen and R₄ is -NH₂.

4. A compound according to claim 1, wherein R₃ is methyl, R₄ is -SH, R₆ is aryl, and R₇ is methyl.

5. A compound according to claim 1, wherein R₂ is hydroxy.

6. A compound according to claim 5, wherein R₃ is hydrogen and R₄ is hydroxy or a protected hydroxy group, R₅ is phenyl or t-butoxy, R₆ is hydrogen, and R₇ is phenyl.

7. A compound according to claim 5, wherein R₃ is hydrogen and R₄ is -NH₂.

8. A compound according to claim 5, wherein R₃ is methyl, and R₄ is -SH.

9. A compound according to claim 5, wherein R₃ and R₆ are hydrogen, R₄ is -NR₈R₉, R₅ is phenyl or t-butoxy, and R₇ is phenyl.

10. A compound according to claim 9, wherein R₈ is hydrogen and R₉ is an amine protecting group.

11. A compound according to any one of claims 1 to 10, wherein R₅ is phenyl.

12. A compound according to any one of claims 1 to 10, wherein R₅ is t-butoxy.

13. A compound according to claim 1, wherein R₂, R₃ and R₆ are hydrogen; R₄ is -NR₈R₉; R₅ is R₁₀ and R₇ is phenyl.

14. A compound according to claim 13, wherein R₈ is hydrogen, R₉ is an amine protecting group and R₁₀ is t-butoxy.

15. The compound 10-desacetoxytaxol.

16. The compound 10-deoxytaxotere.

## Claims (Claims for the following Contracting State(s): ES, GR:)

1. A process for the production of a compound of the formula 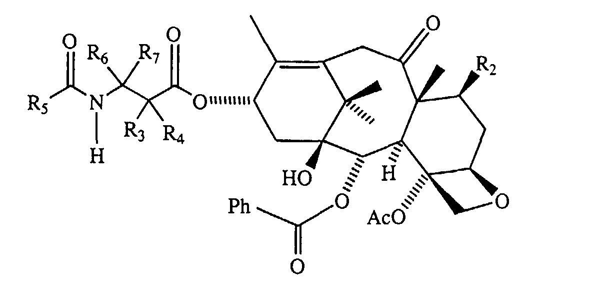 wherein R₂ is hydrogen, hydroxy or a protected hydroxy group; R₃ and R₄ are independently hydrogen, hydroxy, a protected hydroxy group, methyl, -SH, -NH₂, or -NR₈R₉; R₅ is R₁₀, or -OR₁₀; R₆ and R₇ are independently hydrogen, alkyl, or aryl; R₈ and R₉ are independently hydrogen, alkyl, alkenyl, alkynyl, or aryl; and R₁₀ is alkyl, alkenyl, alkynyl, or aryl; provided that when R₂ is hydrogen, R₃ and R₄ are independently hydrogen, methyl, -SH, -NH₂, or -NR₈R₉, which comprises introducing a hydroxy protecting group into 10-deacetyltaxol or a derivative thereof so as to protect the 2'-hydroxy group thereof, removing the 10-hydroxy group from the resulting 2'-hydroxy protected 10-deacetyltaxol or derivative thereof to give a 2'-hydroxy protected intermediate, and then either (i) removing the 2'-hydroxy protecting group from the resulting 2'-hydroxy protected intermediate, and subjecting the resulting 2'-hydroxy compound to catalytic hydrogenation, or (ii) subjecting the 2'-hydroxy protected intermediate to catalytic hydrogenation, and removing the 2'-hydroxy protecting group from the resulting 2'-hydroxy protected compound.

2. A process for the production of a compound of the formula 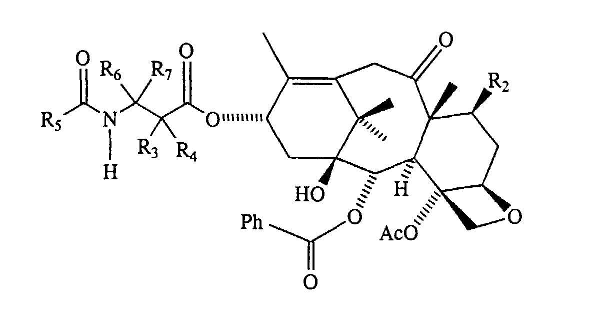 wherein R₂ is hydrogen, hydroxy or a protected hydroxy group; R₃ and R₄ are independently hydrogen, hydroxy, a protected hydroxy group, methyl, -SH, -NH₂, or -NR₈R₉; R₅ is phenyl; R₆ and R₇ are independently hydrogen, alkyl, or aryl; and R₈ and R₉ are independently hydrogen, alkyl, alkenyl, alkynyl, or aryl; provided that when R₂ is hydrogen, R₃ and R₄ are independently hydrogen, methyl, -SH, -NH₂, or -NR₈R₉, which comprises introducing a hydroxy protecting group or groups into 10-deacetyltaxol, 10-deacetyl-7-epitaxol, or a derivative thereof so as to protect both the 2'- and 7-hydroxy groups of 10-deacetyltaxol or derivative thereof or the 2'-hydroxy group of l0-deacetyl-7-epitaxol or derivative thereof, removing the 10-hydroxy group from the resulting 2',7-bishydroxy protected 10-deacetyltaxol, 2'-hydroxy protected 10-deacetyl-7-epitaxol, or derivative thereof to give a 10-desacetoxy intermediate, and then either (i) removing the 2'- and, if present, 7-hydroxy protecting group from the 10-desacetoxy intermediate, and subjecting the resulting 10-desacetoxy compound to catalytic hydrogenation, or (ii) subjecting the 10-desacetoxy intermediate to catalytic hydrogenation, and removing the hydroxy protecting group or groups from the resulting 10-desacetoxy compound.

3. A process for the production of a compound of the formula 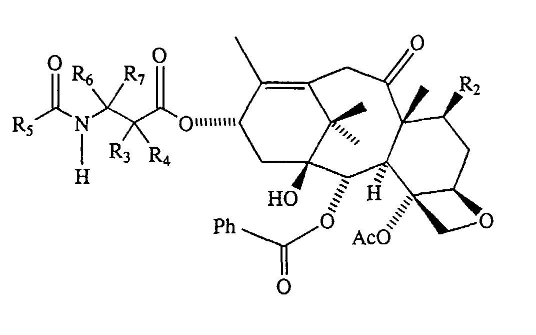 wherein R₂ is hydroxy or a protected hydroxy group; R₃ is hydrogen; R₄ is hydrogen, hydroxy, or a protected hydroxy group; R₅ is t-butoxy; R₆ is hydrogen; R₇ is phenyl; and R₈ and R₉ are independently hydrogen, alkyl, alkenyl, alkynyl, or aryl; which comprises treating 7-hydroxy protected 10-desacetoxy baccatin III, 10-desacetoxy-7-epi-baccatin III, or derivative thereof with a strong base to generate the corresponding 13-alkoxide, reacting the resulting 13-alkoxide with a β-lactam, and removing the protecting groups from the resulting compound.

4. A process for the production of a compound of the formula 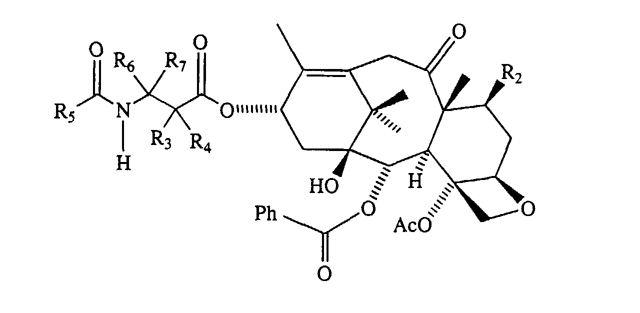 wherein R₂ is hydrogen, hydroxy or a protected hydroxy group; R₃ and R₄ are independently hydrogen, hydroxy, a protected hydroxy group, methyl, -SH, -NH₂, or -NR₈R₉; R₅ is R₁₀, or -OR₁₀; R₆ and R₇ are independently hydrogen, alkyl, or aryl; R₈ and R₉ are independently hydrogen, alkyl, alkenyl, alkynyl, or aryl; and R₁₀ is alkyl, alkenyl, alkynyl, or aryl; provided that when R₂ is hydrogen, R₃ and R₄ are independently hydrogen, methyl, -SH, -NH₂, or -NR₈R₉, which comprises treating 10-deacetyl-baccatin III, 10-deacetyl-7-epibaccatin III, or a derivative thereof with a strong base to generate an alkoxide, forming a thiocarbonate from the alkoxide, and then either (i) reacting the resulting thiocarbonate with a triorganotin hydride in the presence of a radical initiator and removing the hydroxy protecting group from the resulting 7-hydroxy protected compound or (ii) reacting the resulting thiocarbonate with a triorganotin hydride under photochemical conditions, and removing the hydroxy protecting group from the resulting 7-hydroxy protected compound.

5. A process according to any one of claims 1, 2 or 4, wherein R₃ is hydrogen and R₄ is hydroxy or a protected hydroxy group, R₅ is phenyl or t-butoxy, R₆ is hydrogen, and R₇ is phenyl.

6. A process according to any one of claims 1, 2 or 4, wherein R₃ is hydrogen and R₄ is -NH₂.

7. A process according to any one of claims 1, 2 or 4, wherein R₃ is methyl, R₄ is -SH, R₆ is aryl, and R₇ is methyl.

8. A process according to any one of claims 1, 2 or 4, wherein R₂ is hydroxy.

9. A process according to claim 8, wherein R₃ is hydrogen and R₄ is hydroxy or a protected hydroxy group, R₅ is phenyl or t-butoxy, R₆ is hydrogen, and R₇ is phenyl.

10. A process according to claim 8, wherein R₃ is hydrogen and R₄ is -NH₂.

11. A process according to claims 8, wherein R₃ is methyl, and R₄ is -SH.

12. A process according to claim 8, wherein R₃ and R₆ are hydrogen, R₄ is -NR₈R₉, R₅ is phenyl or t-butoxy, and R₇ is phenyl.

13. A process according to claim 12, wherein R₈ is hydrogen and R₉ is an amine protecting group.

14. A process according to any one of claims 1 to 13, wherein R₅ is phenyl.

15. A process according to any one of claims 1 to 13, wherein R₅ is t-butoxy.

16. A process according to any one of claims 1 to 4, wherein R₂, R₃ and R₆ are hydrogen; R₄ is -NR₈R₉; R₅ is R₁₀; and R₇ is phenyl.

17. A process according to claim 16, wherein R₈ is hydrogen, R₉ is an amine protecting group and R₁₀ is t-butoxy.

18. A process according to any one of claims 1 to 17 for the production of 10-desacetoxytaxol.

19. A process according to any one of claims 1 to 17 for the production of 10-deoxytaxotere.

## Patentansprüche (Patentansprüche für folgende(n) Vertragsstaat(en): AT, BE, CH, LI, DE, DK, FR, GB, IE, IT, LU, MC, NL, SE)

1. Verbindung der Formel 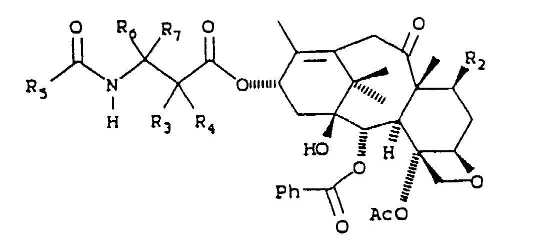 worin R₂ Wasserstoff, Hydroxy oder eine geschützte Hydroxy-Gruppe ist; R₃ und R₄ unabhängig Wasserstoff, Hydroxy, eine geschützte Hydroxy-Gruppe, Methyl, -SH, -NH₂ oder -NR₈R₉ sind; R₅ R₁₀ oder -OR₁₀ ist; R₆ und R₇ unabhängig Wasserstoff, Alkyl oder Aryl sind; R₈ und R₉ unabhängig voneinander Wasserstoff, Alkyl, Alkenyl, Alkynyl oder Aryl sind; und R₁₀ Alkyl, Alkenyl, Alkynyl oder Aryl ist; vorausgesetzt, daß, wenn R₂ Wasserstoff ist, R₃ und R₄ unabhängig Wasserstoff, Methyl, -SH, -NH₂ oder -NR₈R₉ sind.

2. Verbindung nach Anspruch 1, bei der R₃ Wasserstoff ist und R₄ Hydroxy oder eine geschützte Hydroxygruppe ist, R₅ Phenyl oder t-Butoxy ist, R₆ Wasserstoff ist und R₇ Phenyl ist.

3. Verbindung nach Anspruch 1, bei der R₃ Wasserstoff ist und R₄ -NH₂ ist.

4. Verbindung nach Anspruch 1, bei der R₃ Methyl ist, R₄ -SH ist, R₆ Aryl ist und R₇ Methyl ist.

5. Verbindung nach Anspruch 1, bei der R₂ Hydroxy ist.

6. Verbindung nach Anspruch 5, bei der R₃ Wasserstoff ist und R₄ Hydroxy oder eine geschützte Hydroxygruppe ist, R₅ Phenyl oder t-Butoxy ist, R₆ Wasserstoff ist und R₇ Phenyl ist.

7. Verbindung nach Anspruch 5, bei der R₃ Wassertsoff ist und R₄ -NH₂ ist.

8. Verbindung nach Anspruch 5, bei der R₃ Methyl ist und R₄ -SH ist.

9. Verbindung nach Anspruch 5, bei der R₃ und R₆ Wasserstoff sind, R₄ -NR₈R₉ ist, R₅ Phenyl oder t-Butoxy ist und R₇ Phenyl ist.

10. Verbindung nach Anspruch 9, worin R₈ Wasserstoff ist und R₉ eine Amin-Schutzgruppe ist.

11. Verbindung nach einem der Ansprüche 1 bis 10, bei der R₅ Phenyl ist.

12. Verbindung nach einem der Ansprüche 1 bis 10, bei der R₅ t-Butoxy ist.

13. Verbindung nach Anspruch 1, bei der R₂, R₃ und R₆ Wasserstoff sind, R₄ -NR₈R₉ ist, R₅ R₁₀ ist und R₇ Phenyl ist.

14. Verbindung nach Anspruch 13, bei der R₈ Wasserstoff, R₉ eine Amin-Schutzgruppe ist und R₁₀ t-Butoxy ist.

15. Die Verbindung 10-Desacetoxytaxol.

16. Die Verbindung 10-Deoxytaxotere.

## Patentansprüche (Patentansprüche für folgende(n) Vertragsstaat(en): ES, GR)

1. Verfahren zur Herstellung einer Verbindung der Formel 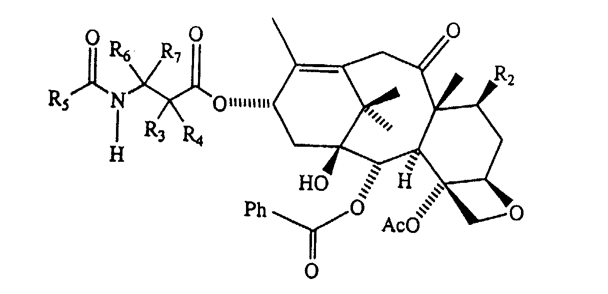 worin R₂ Wasserstoff, Hydroxy oder eine geschützte Hydroxy-Gruppe ist; R₃ und R₄ unabhängig Wasserstoff, Hydroxy, eine geschützte Hydroxy-Gruppe, Methyl, -SH, -NH₂ oder -NR₈R₉ sind; R₅ R₁₀ oder -OR₁₀ ist; R₆ und R₇ unabhängig Wasserstoff, Alkyl oder Aryl sind; R₈ und R₉ unabhängig Wasserstoff, Alkyl, Alkenyl, Alkynyl oder Aryl sind; und R₁₀ Alkyl, Alkenyl, Alkynyl oder Aryl ist; vorausgesetzt daß, wenn R₂ Wasserstoff ist, R₃ und R₄ unabhängig Wasserstoff, Methyl, -SH, -NH₂ oder -NR₈R₉ sind, bei dem man eine Hydroxy-Schutzgruppe in 10-Deacetyltaxol oder ein Derivat davon einführt, um deren 2'-Hydroxygruppe zu schützen, die 10-Hydroxygruppe von dem erhaltenen 2'-Hydroxy-geschützten 10-Deacetyltaxol oder dessen Derivat entfernt, um ein 2'-Hydroxy-geschütztes Zwischenprodukt zu erhalten, und dann entweder (i) die 2'-Hydroxy-Schutzgruppe aus dem erhaltenen 2'-Hydroxy-geschützten Zwischenprodukt entfernt und die erhaltene 2'-Hydroxy-Verbindung katalytisch hydriert, oder (ii) das 2'-Hydroxy-geschützte Zwischenprodukt katalytisch hydriert und die 2'-Hydroxy-Schutzgruppe aus der erhaltenen 2'-Hydroxy-geschützten Verbindung entfernt.

2. Verfahren zur Herstellung einer Verbindung der Formel 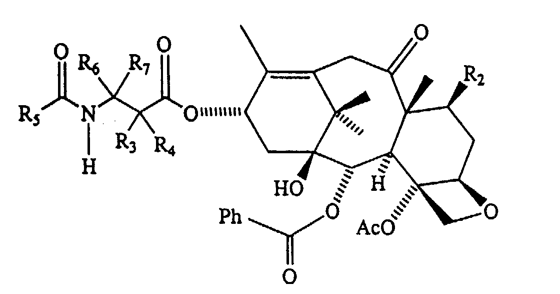 worin R₂ Wasserstoff, Hydroxy oder eine geschützte Hydroxy-Gruppe ist; R₃ und R₄ unabhängig Wasserstoff, Hydroxy, eine geschützte Hydroxy-Gruppe, Methyl, -SH, -NH₂ oder -NR₈R₉ sind; R₅ Phenyl ist; R₆ und R₇ unabhängig Wasserstoff, Alkyl oder Aryl sind; und R₈ und R₉ unabhängig Wasserstoff, Alkyl, Alkenyl, Alkynyl oder Aryl sind; vorausgesetzt, daß, wenn R₂ Wasserstoff ist, R₃ und R₄ unabhängig Wasserstoff, Methyl, -SH, -NH₂ oder -NR₈R₉ sind, bei dem man eine Hydroxy-Schutzgruppe oder -gruppen in 10-Deacetyltaxol, 10-Deacetyl-7-epitaxol oder ein Derivat davon einführt, um die 2'- und 7-Hydroxygruppen des 10-Deacetyltaxols oder seines Derivats oder die 2'-Hydroxy-Gruppe des 10-Deacetyl-7-epitaxols oder seines Derivats zu schützen, die 10-Hydroxy-Gruppe aus dem erhaltenen 2',7-Bishydroxy-geschützten 10-Deacetyltaxol, 2'-Hydroxy-geschützten 10-Deacetyl-7-epitaxol oder dessen Derivat entfernt, um ein 10-Desacetoxy-Zwischenprodukt zu erhalten,, und dann entweder (i) die 2'- und, falls vorhanden, die 7-Hydroxy-Schutzgruppe aus dem 10-Desacetoxy-Zwischenprodukt entfernt und die erhaltene 10-Desacetoxy-Verbindung katalytisch hydriert, oder (ii) das 10-Desacetoxy-Zwischenprodukt katalytisch hydriert und die Hydroxy-Schutzgruppe oder -gruppen aus der entstandenen 10-Desacetoxy-Verbindung entfernt.

3. Verfahren zur Herstellung einer Verbindung der Formel 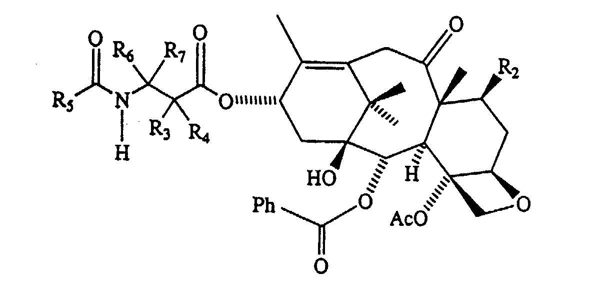 worin R₂ Hydroxy oder eine geschützte Hydroxy-Gruppe ist; R₃ Wasserstoff ist; R₄ Wasserstoff, Hydroxy oder eine geschützte Hydroxy-Gruppe ist; R₅ t-Butoxy ist; R₆ Wasserstoff ist; R₇ Phenyl ist; und R₈ und R₉ unabhängig Wasserstoff, Alkyl, Alkenyl, Alkynyl oder Aryl sind, bei dem man 7-Hydroxy-geschütztes 10-Desacetoxy-baccatin III, 10-Desacetoxy-7-epibaccatin III oder ein Derivat davon mit einer starken Base zur Erzeugung des entsprechenden 13-Alkoxids behandelt, das entstandene 13-Alkoxid mit einem β-Laktam umsetzt und die Schutzgruppen aus der entstandenen Verbindung entfernt.

4. Verfahren zur Herstellung einer Verbindung der Formel 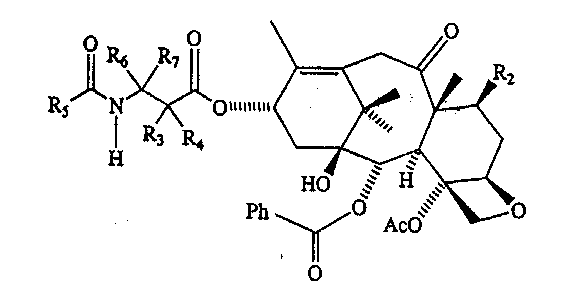 worin R₂ Wasserstoff, Hydroxy oder eine geschützte Hydroxy-Gruppe ist; R₃ und R₄ unabhängig Wasserstoff, Hydroxy, eine geschützte Hydroxy-Gruppe, Methyl, -SH, -NH₂ oder -NR₈R₉ sind; R₅ R₁₀ oder -OR₁₀ ist; R₆ und R₇ unabhängig Wasserstoff, Alkyl oder Aryl sind; R₈ und R₉ unabhängig Wasserstoff, Alkyl, Alkenyl, Alkynyl oder Aryl sind; und R₁₀ Alkyl, Alkenyl, Alkynyl oder Aryl sind, vorausgesetzt, daß, wenn R₂ Wasserstoff ist, R₃ und R₄ unabhängig Wasserstoff, Methyl, -SH, -NH₂ oder -NR₈R₉ sind, bei dem man 10-Deacetyl-baccatin III, 10-Deacetyl-7-epibaccatin III oder ein Derivat davon mit einer starken Base zur Bildung eines Alkoxids behandelt, aus dem Alkoxid ein Thiocarbonat bildet, und dann entweder (i) das entstandene Thiocarbonat in Gegenwart eines Radikalinitiators mit einem Triorganozinnhydrid umsetzt und die Hydroxy-Schutzgruppe aus der entstandenen 7-Hydroxy-geschützten Verbindung entfernt, oder (ii) das entstandene Thiocarbonat unter photochemischen Bedingungen mit einem Triorganozinnhydrid umsetzt und die Hydroxy-geschützte Gruppe aus der entstandenen 7-Hydroxy-geschützten Verbindung entfernt.

5. Verfahren nach einem der Ansprüche 1,2 oder 4, bei dem R₃ Wasserstoff ist und R₄ Hydroxy oder eine geschützte Hydroxy-Gruppe ist, R₅ Phenyl oder t-Butoxy ist, R₆ Wasserstoff ist und R₇ Phenyl ist.

6. Verfahren nach einem der Ansprüche 1,2 oder 4, bei dem R₃ Wasserstoff ist und R₄ -NH₂ ist.

7. Verfahren nach einem der Ansprüche 1, 2 oder 4, bei dem R₃ Methyl ist, R₄ -SH ist, R₆ Aryl ist und R₇ Methyl ist.

8. Verfahren nach einem der Ansprüche 1,2 oder 4, bei dem R₂ Hydroxy ist.

9. Verfahren nach Anspruch 8, bei dem R₃ Wasserstof ist und R₄ Hydroxy oder eine geschützte Hydroxy-Gruppe ist, R₅ Phenyl oder t-Butoxy ist, R₆ Wasserstoff ist und R₇ Phenyl ist.

10. Verfahren nach Anspruch 8, bei dem R₃ Wasserstoff ist und R₄ -NH₂ ist.

11. Verfahren nach Anspruch 8, bei dem R₃ Methyl ist und R₄ -SH ist.

12. Verfahren nach Anspruch 8, bei dem R₃ und R₆ Wasserstoff sind, R₄ -NR₈R₉ ist, R₅ Phenyl oder t-Butoxy ist und R₇ Phenyl ist.

13. Verfahren nach Anspruch 12, bei dem R₈ Wasserstoff ist und R₉ eine Amin-Schutzgruppe ist.

14. Verfahren nach einem der Ansprüche 1 bis 13, bei dem R₅ Phenyl ist.

15. Verfahren nach einem der Ansprüche 1 bis 13, bei dem R₅ t-Butoxy ist.

16. Verfahren nach einem der Ansprüche 1 bis 4, bei dem R₂, R₃ und R₆ Wasserstoff sind, R₄ -NR₈R₉ ist, R₅ gleich R₁₀ ist und R₇ Phenyl ist.

17. Verfahren nach Anspruch 16, bei dem R₈ Wasserstoff ist, R₉ eine Amin-Schutzgruppe ist und R₁₀ t-Butoxy ist.

18. Verfahren nach einem der Ansprüche 1 bis 17 zur Herstellung von 10-Desacetoxytaxol.

19. Verfahren nach einem der Ansprüche 1 bis 17 zur Herstellung von 10-Deoxytaxotere.

## Revendications (Revendications pour l'(les) Etat(s) contractant(s) suivant(s): AT, BE, CH, LI, DE, DK, FR, GB, IE, IT, LU, MC, NL, SE)

1. Composé de la formule 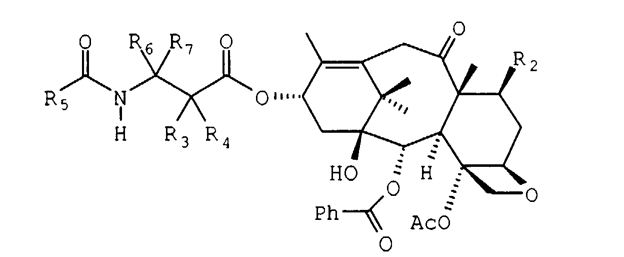 dans laquelle R₂ est hydrogène, hydroxy ou un groupe hydroxy protégé ; R₃ et R₄ sont indépendamment l'un de l'autre hydrogène, hydroxy, un groupe hydroxy protégé, méthyle, -SH, -NH₂ ou -NR₈R₉ ; R₅ est R₁₀ ou -OR₁₀ ; R₆ et R₇ sont indépendamment l'un de l'autre hydrogène, alkyle ou aryle ; R₈ et R₉ sont indépendamment l'un de l'autre hydrogène, alkyle, alcényle, alcynyle ou aryle ; et R₁₀ est alkyle, alcényle, alcynyle ou aryle ; à la condition que, lorsque R₂ est hydrogène, R₃ et R₄ sont indépendamment l'un de l'autre hydrogène, méthyle, -SH, -NH₂ ou -NR₈R₉.

2. Composé selon la revendication 1, dans lequel R₃ est hydrogène et R₄ est hydroxy ou un groupe hydroxy protégé, R₅ est phényle ou t-butoxy, R₆ est hydrogène et R₇ est phényle.

3. Composé selon la revendication 1, dans lequel R₃ est hydrogène et R₄ est -NH₂.

4. Composé selon la revendication 1, dans lequel R₃ est méthyle, R₄ est -SH, R₆ est aryle et R₇ est méthyle.

5. Composé selon la revendication 1, dans lequel R₂ est hydroxy.

6. Composé selon la revendication 5, dans lequel R₃ est hydrogène et R₄ est hydroxy ou un groupe hydroxy protégé, R₅ est phényle ou t-butoxy, R₆ est hydrogène et R₇ est phényle.

7. Composé selon la revendication 5, dans lequel R₃ est hydrogène et R₄ est -NH₂.

8. Composé selon la revendication 5, dans lequel R₃ est méthyle et R₄ est -SH.

9. Composé selon la revendication 5, dans lequel R₃ et R₆ sont hydrogène, R₄ est -NR₈R₉, R₅ est phényle ou t-butoxy et R₇ est phényle.

10. Composé selon la revendication 9, dans lequel R₈ est hydrogène et R₉ est un groupe aminoprotecteur.

11. Composé selon l'une quelconque des revendications 1 à 10, dans lequel R₅ est phényle.

12. Composé selon l'une quelconque des revendications 1 à 10, dans lequel R₅ est t-butoxy.

13. Composé selon la revendication 1, dans lequel R₂, R₃ et R₆ sont hydrogène, R₄ est -NR₈R₉, R₅ est R₁₀ et R₇ est phényle.

14. Composé selon la revendication 13, dans lequel R₈ est hydrogène, R₉ est un groupe aminoprotecteur et R₁₀ est t-butoxy.

15. Le composé 10-désacétoxytaxol.

16. Le composé 10-désoxytaxotère.

## Revendications (Revendications pour l'(les) Etat(s) contractant(s) suivant(s): ES, GR)

1. Procédé de production d'un composé de la formule 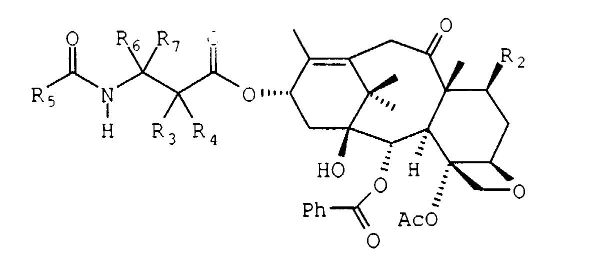 dans laquelle R₂ est hydrogène, hydroxy ou un groupe hydroxy protégé ; R₃ et R₄ sont indépendamment l'un de l'autre hydrogène, hydroxy, un groupe hydroxy protégé, méthyle, -SH, -NH₂ ou -NR₈R₉ ; R₅ est R₁₀ ou -OR₁₀ ; R₆ et R₇ sont indépendamment l'un de l'autre hydrogène, alkyle ou aryle ; R₈ et R₉ sont indépendamment l'un de l'autre hydrogène, alkyle, alcényle, alcynyle ou aryle ; et R₁₀ est alkyle, alcényle, alcynyle ou aryle ; à la condition que, lorsque R₂ est hydrogène, R₃ et R₄ sont indépendamment l'un de l'autre hydrogène, méthyle, -SH, -NH₂ ou -NR₈R₉ ; ledit procédé comprenant les étapes consistant à introduire un groupe hydroxyprotecteur dans le 10-désacétyltaxol ou un dérivé de celui-ci afin de protéger le groupe 2'-hydroxy de celui-ci ; enlever le groupe 10-hydroxy du 10-désacétyltaxol ou dérivé de celui-ci 2'-hydroxyprotégé résultant afin d'obtenir un intermédiaire 2'-hydroxyprotégé ; et ensuite soit (i) enlever le groupe 2'-hydroxyprotecteur de l'intermédiaire 2'-hydroxyprotégé résultant et soumettre le composé 2'-hydroxy résultant à une hydrogénation catalytique, soit (ii) soumettre l'intermédiaire 2'-hydroxyprotégé à une hydrogénation catalytique et enlever le groupe 2'-hydroxy protecteur du composé 2'-hydroxy résultant.

2. Procédé de production d'un composé de la formule 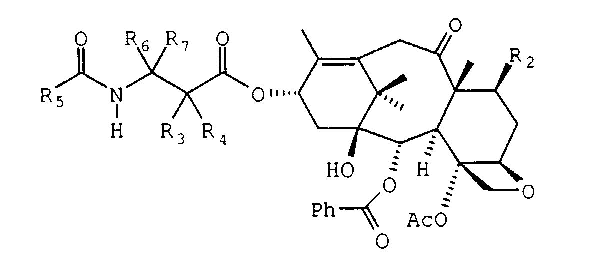 dans laquelle R₂ est hydrogène, hydroxy ou un groupe hydroxy protégé ; R₃ et R₄ sont indépendamment l'un de l'autre hydrogène, hydroxy, un groupe hydroxy protégé, méthyle, -SH, -NH₂ ou -NR₈R₉ ; R₅ est phényle ; R₆ et R₇ sont indépendamment l'un de l'autre hydrogène, alkyle ou aryle ; et R₈ et R₉ sont indépendamment l'un de l'autre hydrogène, alkyle, alcényle, alcynyle ou aryle ; à la condition que, lorsque R₂ est hydrogène, R₃ et R₄ sont indépendamment l'un de l'autre hydrogène, méthyle, -SH, -NH₂ ou -NR₈R₉ ; ledit procédé comprenant les étapes consistant à introduire un ou des groupes hydroxyprotecteurs dans le 10-désacétyltaxol, le 10-désacétyl-7-épitaxol ou un dérivé de ceux-ci afin de protéger à la fois les groupes 2'- et 7-hydroxy du 10-désacétyltaxol ou dérivé de celui-ci ou bien le groupe 2'-hydroxy du 10-désacétyl-7-épitaxol ou dérivé de celui-ci ; enlever le groupe 10-hydroxy du 10-désacétyltaxol 2',7-bishydroxy-protégé, du 10-désacétyl-7-épitaxol 2'-hydroxyprotégé ou des dérivés de ceux-ci résultants afin d'obtenir un intermédiaire 10-désacétoxy ; et ensuite soit (i) enlever le groupe 2'-hydroxyprotecteur et, s'il est présent, le groupe 7-hydroxyprotecteur de l'intermédiaire 10-désacétoxy résultant et soumettre le composé 10-désacétoxy résultant à une hydrogénation catalytique, soit (ii) soumettre l'intermédiaire 10-désacétoxy à une hydrogénation catalytique et enlever le ou les groupes hydroxyprotecteurs du composé 10-désacétoxy résultant.

3. Procédé de production d'un composé de la formule 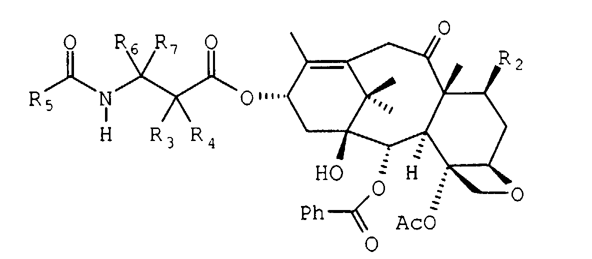 dans laquelle R₂ est hydroxy ou un groupe hydroxy protégé ; R₃ est hydrogène ; R₄ est hydrogène, hydroxy, un groupe hydroxy protégé ; R₅ est t-butoxy ; R₆ est hydrogène ; R₇ est phényle ; et R₈ et R₉ sont indépendamment l'un de l'autre hydrogène, alkyle, alcényle, alcynyle ou aryle ; ledit procédé comprenant les étapes consistant à traiter la 7-hydroxyprotégé 10-désacétoxybaccatine III, la 10-désacétoxy-7-épibaccatine III ou un dérivé de celles-ci avec une base forte afin de générer le 13-alcoolate correspondant ; faire réagir le 13-alcoolate résultant avec un β-lactame ; et enlever les groupes protecteurs du composé résultant.

4. Procédé de production d'un composé de la formule 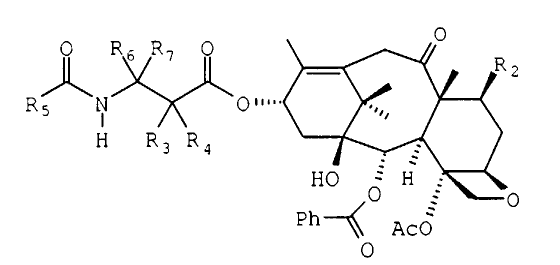 dans laquelle R₂ est hydrogène, hydroxy ou un groupe hydroxy protégé ; R₃ et R₄ sont indépendamment l'un de l'autre hydrogène, hydroxy, un groupe hydroxy protégé, méthyle, -SH, -NH₂ ou -NR₈R₉ ; R₅ est R₁₀ ou -OR₁₀ ; R₆ et R₇ sont indépendamment l'un de l'autre hydrogène, alkyle, ou aryle ; R₈ et R₉ sont indépendamment l'un de l'autre hydrogène, alkyle, alcényle, alcynyle ou aryle ; et R₁₀ est alkyle, alcényle, alcynyle ou aryle ; à la condition que, lorsque R₂ est hydrogène, R₃ et R₄ sont indépendamment l'un de l'autre hydrogène, méthyle, -SH, -NH₂ ou -NR₈R₉ ; ledit procédé comprenant les étapes consistant à traiter la 10-désacétylbaccatine III, la 10-désacétyl-7-épibaccatine III ou un dérivé de celles-ci avec une base forte afin de générer un alcoolate ; former un thiocarbonate à partir de l'alcoolate ; et ensuite soit (i) faire réagir le thiocarbonate résultant avec un hydrure de triorganoétain en présence d'un amorceur radicalaire et enlever les groupes hydroxyprotecteurs du composé 7-hydroxyprotégé résultant, soit (ii) faire réagir le thiocarbonate résultant avec un hydrure de triorganoétain en conditions photochimiques et enlever les groupes hydroxyprotecteurs du composé 7-hydroxyprotégé résultant.

5. Procédé selon l'une quelconque des revendications 1, 2 ou 4, dans lequel R₃ est hydrogène et R₄ est hydroxy ou un groupe hydroxy protégé, R₅ est phényle ou t-butoxy, R₆ est hydrogène et R₇ est phényle.

6. Procédé selon l'une quelconque des revendications 1, 2 ou 4, dans lequel R₃ est hydrogène et R₄ est -NH₂.

7. Procédé selon l'une quelconque des revendications 1, 2 ou 4, dans lequel R₃ est méthyle, R₄ est -SH, R₆ est aryle et R₇ est méthyle.

8. Procédé selon l'une quelconque des revendications 1, 2 ou 4, dans lequel R₂ est hydroxy.

9. Procédé selon la revendication 8, dans lequel R₃ est hydrogène et R₄ est hydroxy ou un groupe hydroxy protégé, R₅ est phényle ou t-butoxy, R₆ est hydrogène et R₇ est phényle.

10. Procédé selon la revendication 8, dans lequel R₃ est hydrogène et R₄ est -NH₂.

11. Procédé selon la revendication 8, dans lequel R₃ est méthyle et R₄ est -SH.

12. Procédé selon la revendication 8, dans lequel R₃ et R₆ sont hydrogène, R₄ est -NR₈R₉, R₅ est phényle ou t-butoxy et R₇ est phényle.

13. Procédé selon la revendication 12, dans lequel R₈ est hydrogène et R₉ est un groupe aminoprotecteur.

14. Procédé selon l'une quelconque des revendications 1 à 13, dans lequel R₅ est phényle.

15. Procédé selon l'une quelconque des revendications 1 à 13, dans lequel R₅ est t-butoxy.

16. Procédé selon l'une quelconque des revendications 1 à 4, dans lequel R₂, R₃ et R₆ sont hydrogène, R₄ est -NR₈R₉, R₅ est R₁₀ et R₇ est phényle.

17. Procédé selon la revendication 16, dans lequel R₈ est hydrogène, R₉ est un groupe aminoprotecteur et R₁₀ est t-butoxy.

18. Procédé selon l'une quelconque des revendications 1 à 17 pour la production de 10-désacétoxytaxol.

19. Procédé selon l'une quelconque des revendications 1 à 17 pour la production de 10-désoxytaxotère.
